# EUROPEAN PATENT APPLICATION

(11) **EP 4 574 994 A1**
(43) Date of publication of application: **25.06.2025**
(21) Application number: 24219163.3
(22) Date of filing: 11.12.2024
(51) Int. Cl.: C12Q 1/6837

(54) **SPATIAL CHIP, METHOD FOR PREPARING SAME, AND USE THEREOF**

(30) Priority: 18.12.2023 CN 202311744836; 06.02.2024 CN 202410168779; 14.08.2024 CN 202411113338
(71) Applicant: GeneMind Biosciences Company Limited, Shenzhen City, 518023 Guangdong (CN)
(72) Inventor: LIU, Lei, Shenzhen City, 518023 (CN); LI, Zhao, Shenzhen City, 518023 (CN); LI, Guang, Shenzhen City, 518023 (CN); LIN, Zhifeng, Shenzhen City, 518023 (CN); WANG, Wen, Shenzhen City, 518023 (CN); CHANG, Chunhui, Shenzhen City, 518023 (CN); YANG, Chunyou, Shenzhen City, 518023 (CN); ZHAN, Naiqian, Shenzhen City, 518023 (CN); CHEN, Fang, Shenzhen City, 518023 (CN); SUN, Lei, Shenzhen City, 518023 (CN)
(74) Representative: Schiweck Weinzierl Koch Patentanwälte Partnerschaft mbB

(57) **Abstract**

The present application relates to the field of biological technology, and provides a spatial chip, a method for preparing same, and use thereof. The spatial chip comprises a chip substrate comprising a first surface provided with a plurality of isolated single-stranded nucleic acid amplification clusters; each of the single-stranded nucleic acid amplification clusters comprises a plurality of single-stranded nucleic acid molecules with identical nucleotide sequences, and nucleotide sequences of different single-stranded nucleic acid amplification clusters are different; each of the single-stranded nucleic acid amplification clusters has a known physical coordinate set relative to the spatial chip; the single-stranded nucleic acid molecule comprises at least a barcode sequence with a known sequence, and the barcode sequences of each single-stranded nucleic acid amplification clusters are in one-to-one correspondence with the physical coordinate sets.

## Description

### TECHNICAL FIELD

The present disclosure relates to the field of biotechnology, and particularly, to a spatial chip, a method for preparing same, and use thereof.

### BACKGROUND

Spatial omics is a research method combining omics and spatial information. In omics, the research object can be biomolecules such as genes, proteins, metabolites, etc., while spatial information relates to the distribution and interaction of such molecules in cells, tissues, and organs. The spatial omics technology can simultaneously acquire the spatial positional information and multiomics data for studies. By analyzing the high-dimensional data, the structure and functionality of the biological system can be interpreted, thus providing an important research means for a variety of fields such as tissue and cell functionality, microenvironment interaction, development lineage tracing, pathology, and the like, as well as a new thought and method for disease diagnosis and treatment and the research and development of drugs. However, there is no spatial chip with universality and convenience in studies. It is thus very important to develop a universal spatial omics chip that can be used for studies on transcriptome, proteome, ATAC (assay for targeting accessible-chromatin) sequencing, etc.

### SUMMARY

The examples of the present application are intended to provide a spatial chip and a method for preparing same, so as to form single-stranded nucleic acid amplification clusters with a barcode sequence and a physical coordinate set on the surface of the spatial chip. The barcode sequences and the physical coordinate sets of the single-stranded nucleic acid amplification cluster are in one-to-one correspondence, such that when the chip is used as a spatial chip, the analyses of various omics including genome, reverse transcriptome, proteome, and the like can be conducted through known site information (including the physical coordinate sets and the barcode sequence corresponding to each position), and the spatial chip is thus suitable for the space information analysis in various omics fields.

The examples of the present application are also intended to provide use of a spatial chip in the field of spatial omics, a composition comprising the spatial chip, a method for spatial reverse transcription, a method for ATAC sequencing, and a method for spatial protein analysis.

To achieve the purpose of the present application described above, the examples of the present application utilize the following technical schemes:
1. A spatial chip, comprising a chip substrate, wherein the chip substrate comprises a first surface provided with a plurality of isolated single-stranded nucleic acid amplification clusters, each of the single-stranded nucleic acid amplification clusters comprises a plurality of single-stranded nucleic acid molecules with identical nucleotide sequences, and nucleotide sequences of different single-stranded nucleic acid amplification clusters are different;
   each of the single-stranded nucleic acid amplification clusters has a known physical coordinate set relative to the spatial chip; the single-stranded nucleic acid molecule comprises at least a barcode sequence with a known sequence, and the barcode sequences of each single-stranded nucleic acid amplification clusters are in one-to-one correspondence with the physical coordinate sets.
2. The spatial chip according to item 1, wherein the number of the amplification clusters on the chip substrate is n, the number of nucleotides constituting the barcode sequence is m, and n and m satisfy: 4^{m} ≥ n; optionally, n is a natural number greater than or equal to 28.
3. The spatial chip according to item 1, wherein in each of the single-stranded nucleic acid amplification clusters, the copy number of the single-stranded nucleic acid molecules is 10-10⁵;
   optionally, the area of each of the single-stranded nucleic acid amplification clusters on the first surface is 0.25-100 µm²;
   optionally, the distance between adjacent single-stranded nucleic acid amplification clusters is 0.1-10 µm.
4. The spatial chip according to any one of items 1-3, wherein the single-stranded nucleic acid molecule further comprises a first capture sequence, and the barcode sequence is located at one end of the first capture sequence; optionally, the first capture sequence is linked to the chip substrate;
   optionally, the first capture sequence is linked to the chip substrate by chemical bonding, physical adsorption, electrostatic adsorption, or Van der Waals force;
   optionally, a first spacer sequence region is arranged between the first capture sequence and the barcode sequence.
5. The spatial chip according to item 4, wherein a compound is bonded to the chip substrate, and the first capture sequence is linked to the chip substrate via the compound;
   optionally, the chip substrate is provided with a first group, the compound is provided with a second group, the first group and the second group are a chemically reactive group set, and the compound is bonded to the chip substrate via the group set;
   optionally, the group set is selected from an amino-carboxylic acid set, an amino-ester set, an amino-halogen set, an amino-formyl set, an amino-epoxy group set, a sulfydryl-imino set, an azido-alkynyl set, an azido-alkenyl set, and a cycloalkenyl-tetrazinyl set;
   optionally, the compound is selected from one or more of an amino compound, a carboxylate compound, succinimide or an analog thereof, a pentafluorophenol ester or an analog thereof, an aryl halide, maleimide or an analog thereof, an epoxy compound, a formyl-containing compound, an azido-containing compound, norbornene or an analog thereof, a DBCO or an analog thereof, and a tetrazine and an analog thereof.
6. The spatial chip according to item 4, wherein the chip substrate is provided with a first reactant, the first capture sequence is provided with a second reactant, the first reactant and the second reactant are reactive, and the first capture sequence is linked to the chip substrate via the first reactant and the second reactant; optionally, the first reactant is different from the second reactant, and the first reactant and the second reactant are each independently selected from an antigen and an antibody, or the first reactant and the second reactant are each independently selected from biotin and streptavidin.
7. The spatial chip according to item 4, wherein the single-stranded nucleic acid molecule further comprises a second capture sequence, and the second capture sequence is linked to the barcode sequence at the end distal to the first capture sequence;
   optionally, a PolyN sequence is arranged between the second capture sequence and the barcode sequence, and N is selected from one of A or a derivative thereof, T/U or a derivative thereof, G or a derivative thereof, and C or a derivative thereof;
   optionally, a second spacer sequence region is arranged between the second capture sequence and the barcode sequence.
8. The spatial chip according to item 7, wherein a cleavable site is arranged in the first capture sequence and the second capture sequence;
   optionally, the cleavable site is at least one of a photocleavable site, a chemically cleavable site, or an enzyme-cleavable site;
   optionally, when the cleavable site is an enzyme-cleavable site, the barcode sequence does not comprise a sequence identical to the cleavable site.
9. The spatial chip according to any one of items 1-8, wherein the chip substrate has a planar surface, a spherical surface, or an irregular surface;
   optionally, the chip substrate is provided with a plurality of micro/nano structures arranged according to a preset rule, and the single-stranded nucleic acid amplification cluster is immobilized on a surface of the micro/nano structure;
   optionally, the micro/nano structure is a nano depression and/or a nano protrusion;
   optionally, the micro/nano structure has a circular, elliptical, polygonal, or irregular cross-sectional profile; optionally, the minimum distance between adjacent micro/nano structures is 10 nm to 100 µm, and the maximum size of a shape of the micro/nano structure on a surface of the chip substrate is 10 nm to 100 µm.
10. The spatial chip according to any one of items 1-8, wherein the first surface is provided with one or more markers;
   optionally, the marker is one of a letter, a number and a graphic, or a combination thereof;
   optionally, the marker includes a directional marker and a regional marker;
   optionally, a plurality of regional markers are present and arranged on the first surface and close to a circumference of the chip substrate;
   optionally, the chip substrate has a polygonal shape, and the regional markers are uniformly distributed in a direction parallel to each side of the polygon; or
   the chip substrate is circular or elliptical, and the regional markers are uniformly distributed along a track close to the circumference;
   optionally, the marker further includes a chip serial number marker.
11. The spatial chip according to item 10, wherein the chip substrate comprises a second surface arranged opposite to the first surface;
   optionally, the second surface is provided with a chip label; the chip label is configured for recording the physical coordinate set of each of the amplification clusters on the chip substrate and a sequence of the barcode sequence region at each of the physical coordinate sets;
   optionally, the chip label is a QR code or a barcode.
12. The spatial chip according to any one of items 1-11, wherein the spatial chip comprises two or more chip substrates, and the chip substrates are immobilized on a surface of a solid carrier in a preset arrangement; optionally, the chip substrate has known positional information relative to the solid carrier;
   optionally, a surface of the solid carrier is provided with a chip label; the chip label is configured for recording the physical coordinate set of each of the amplification clusters on the chip substrate and a sequence of the barcode sequence region at each of the physical coordinate sets;
   optionally, the positional information is acquired by the chip serial number marker of the first surface; optionally, a glue layer is arranged between the chip substrate and the solid carrier;
   optionally, the glue layer covers part or all of the second surface;
   optionally, the glue layer has a light transmittance of greater than or equal to 90%;
   optionally, the chip substrate is arrayed on the surface of the solid carrier;
   optionally, the distance between the circumference of the chip substrate and the circumference of the solid carrier is greater than or equal to 1 mm;
   optionally, the distance between adjacent chip substrates is greater than or equal to 1 mm;
   optionally, the solid carrier has a light transmittance of greater than or equal to 90%.
13. A method for preparing a spatial chip, comprising:
   synthesizing a first single-stranded nucleotide library with a barcode sequence, wherein nucleotide sequences of different first single-stranded nucleotide libraries are different, and the barcode sequences of the first single-stranded nucleotide libraries are different from each other;
   immobilizing the first single-stranded nucleotide library on a surface of a solid substrate, and amplifying the first single-stranded nucleotide library to give an amplification cluster;
   linearizing the amplification cluster to give a single-stranded nucleic acid amplification cluster; and
   sequencing the single-stranded nucleic acid amplification cluster to acquire the barcode sequence of each of the single-stranded nucleic acid amplification clusters and a physical coordinate set of each of the single-stranded nucleic acid amplification clusters on a surface of the chip substrate, and determining information that the barcode sequences of the first single-stranded nucleotide library are in one-to-one correspondence with the physical coordinate sets, so as to give the spatial chip.
14. The method according to item 13, wherein the number of the amplification clusters on the surface of the solid substrate is n, the number of nucleotides constituting the barcode sequence is m, and n and m satisfy: 4^{m} ≥ n;
   optionally, n is a natural number greater than or equal to 28;
   optionally, the area of each of the amplification clusters is 0.25-100 µm²;
   optionally, in each of the amplification clusters, the copy number of the nucleic acid molecules is 10-10⁵;
   optionally, the distance between adjacent nucleic acid amplification clusters is 0.1-10 µm.
15. The method according to item 14, wherein the first single-stranded nucleotide library further comprises a first capture sequence, and the barcode sequence is located at one end of the first capture sequence; optionally, in the step of immobilizing the first single-stranded nucleotide library on the surface of the solid substrate, the first capture sequence is bonded to the surface of the solid substrate;
   optionally, the first capture sequence is linked to the surface of the chip substrate by chemical bonding, physical adsorption, electrostatic adsorption, or Van der Waals force;
   optionally, a compound is bonded to the surface of the solid substrate, and the first capture sequence is linked to the chip substrate via the compound;
   optionally, the surface of the solid substrate is provided with a first group, the compound is provided with a second group, the first group and the second group are a chemically reactive group set, and the compound is bonded to the surface of the chip substrate via the group set;
   optionally, the group set is selected from an amino-carboxylic acid set, an amino-ester set, an amino-halogen set, an amino-formyl set, an amino-epoxy group set, a sulfydryl-imino set, an azido-alkynyl set, an azido-alkenyl set, and a cycloalkenyl-tetrazinyl set;
   optionally, the compound is selected from one or more of an amino compound, a carboxylate compound, succinimide or an analog thereof, a pentafluorophenol ester or an analog thereof, an aryl halide, maleimide or an analog thereof, an epoxy compound, a formyl-containing compound, an azido-containing compound, norbornene or an analog thereof, a DBCO or an analog thereof, and a tetrazine and an analog thereof;
   optionally, the surface of the solid substrate is provided with a first reactant, the first capture sequence is provided with a second reactant, the first reactant and the second reactant are reactive, and the first capture sequence is linked to the surface of the solid substrate via the first reactant and the second reactant;
   optionally, the first reactant is different from the second reactant, and the first reactant and the second reactant are each independently selected from an antigen and an antibody, or the first reactant and the second reactant are each independently selected from biotin and streptavidin;
   optionally, the first single-stranded nucleotide library further comprises a second capture sequence, and the second capture sequence is linked to the barcode sequence at the end distal to the first capture sequence; optionally, a PolyN sequence is arranged between the second capture sequence and the barcode sequence, and N is selected from one of A or a derivative thereof, T/U or a derivative thereof, G or a derivative thereof, and C or a derivative thereof;
   optionally, a cleavable site is arranged in the first capture sequence and the second capture sequence; optionally, the cleavable site is at least one of a photocleavable site, a chemically cleavable site, or an enzyme-cleavable site;
   optionally, when the cleavable site is an enzyme-cleavable site, the barcode sequence does not comprise a sequence identical to the cleavable site;
   optionally, the step of linearizing the amplification cluster comprises: adding a restriction endonuclease, and digesting the amplification cluster at an enzyme-cleavable site.
16. The method according to any one of items 13-15, wherein the surface of the solid substrate is a planar surface, a spherical surface, or a non-planar surface formed by a dendrimer;
   optionally, the surface of the solid substrate is provided with a nanowell or a nanopore, and the first single-stranded nucleotide library is immobilized in the nanowell or the nanopore.
17. The method according to any one of items 13-16, wherein the solid substrate is derived from a chip mother substrate having an area 2 or more times a surface area of the spatial chip, and the method further comprises, before the spatial chip is given: cutting a surface of the chip mother substrate into a chip substrate with a preset size;
   optionally, the step of cutting a surface of the chip mother substrate into a chip substrate with a preset size comprises:
   determining one or more preset chip regions on the surface of the chip mother substrate according to a preset shape and the preset size, and marking the preset chip region;
   separating the preset chip region from the chip mother substrate to give the spatial chip, wherein the spatial chip comprises the chip substrate, the chip substrate is provided with a first surface and a second surface opposite to the first surface, and the single-stranded nucleic acid amplification cluster is located on the first surface;
   optionally, the method further comprises: fixing one or more chip substrates on a surface of a solid carrier in a preset arrangement.
18. The method according to item 17, wherein marking the preset chip region comprises:
   printing a marker on a surface of the preset chip region by laser printing;
   optionally, the marking comprises forming the marker on the first surface;
   optionally, the marker includes a directional marker, a regional marker, and a chip serial number marker;
   optionally, a plurality of regional markers are present and arranged on the first surface and close to a circumference of the first substrate;
   optionally, the marker is one of a letter, a number and a graphic, or a combination thereof;
   optionally, separating the preset chip region from the chip mother substrate comprises:
      cutting and separating the preset chip region from the chip mother substrate by laser according to a preset cutting line;
      optionally, the distance between adjacent preset cutting lines is greater than or equal to 2 mm;
      optionally, the method further comprises:
      fixing the chip substrate on one surface of the solid carrier;
      optionally, a surface of the solid carrier is provided with a chip label; the chip label is configured for recording the physical coordinate set of each of the amplification clusters on the chip substrate and a sequence of the barcode sequence region at each of the physical coordinate sets; or alternatively, the second surface is provided with a chip label; the chip label is configured for recording the physical coordinate set of each of the amplification clusters on the chip substrate and a sequence of the barcode sequence region at each of the
      physical coordinate sets;
      optionally, the chip label is a QR code or a barcode;
      optionally, the second surface is bonded to the solid carrier, and the chip substrate has known positional information relative to the solid carrier;
      optionally, a glue layer is arranged between the chip substrate and the solid carrier;
      optionally, the glue layer covers part or all of the second surface;
      optionally, the glue layer has a light transmittance of greater than or equal to 90%;
      optionally, the chip substrate is arrayed on the solid carrier;
      optionally, the distance between the circumference of the chip substrate and the circumference of the solid carrier is greater than or equal to 1 mm;
      optionally, the distance between adjacent chip substrates is greater than or equal to 1 mm.
19. Use of the spatial chip according to any one of items 1-12 or a spatial chip prepared by the method according to any one of items 13-18 in the field of spatial omics.
20. The use according to item 19, wherein the use is a method for spatial reverse transcription comprising:
   providing the spatial chip and a second single-stranded nucleotide library, wherein the spatial chip comprises a chip substrate; at least one surface of the chip substrate is provided with a plurality of isolated single-stranded nucleic acid amplification clusters; single-stranded nucleic acid molecules in the single-stranded nucleic acid amplification cluster have identical nucleotide sequences, and nucleotide sequences of different single-stranded nucleic acid amplification clusters are different; the single-stranded nucleic acid amplification cluster has a known physical coordinate set relative to the spatial chip, and the single-stranded nucleic acid molecule comprises a barcode sequence with a known sequence and a first capture sequence and a second capture sequence respectively linked to the two ends of the barcode sequence; the first capture sequence is linked to a surface of the chip substrate, and the barcode sequences of each single-stranded nucleic acid amplification clusters are in one-to-one correspondence with the physical coordinate sets; the second single-stranded nucleotide library comprises a first base sequence, a second base sequence and a capture part that are linked in sequence; the first base sequence is a known sequence complementary with the second capture sequence, the second base sequence is a label sequence, the capture part is a primer sequence, and first base sequences of the nucleic acid in the second single-stranded nucleotide library are identical, but the label sequences are different;
   contacting a cell or tissue sample of interest with the spatial chip and the second single-stranded nucleotide library to bond RNA in the cell or tissue sample of interest on the spatial chip via the second single-stranded nucleotide library;
   cleaving the spatial chip, and collecting a cleavage product; and
   sequencing the cleavage product, and analyzing obtained sequencing data;
   optionally, when the cell or tissue sample of interest is a tissue sample, the tissue sample is a tissue section;
   optionally, the tissue section has a thickness of less than or equal to 50 µm;
   optionally, the step of contacting the cell or tissue sample of interest with the spatial chip and the second single-stranded nucleotide library comprises:
      contacting the cell or tissue sample of interest with the spatial chip, and then adding the second single-stranded nucleotide library;
      optionally, analyzing the obtained sequencing data comprises:
         on the basis of the sequencing data, acquiring positional information in the spatial chip of the barcode sequence in the cleavage product; and
         on the basis of the positional information, performing spatial restoration on the cleavage product to achieve spatial reverse transcription analysis.
21. The use according to item 19, wherein the use is a method for ATAC sequencing comprising:
   providing the spatial chip and a second single-stranded nucleotide library, wherein the spatial chip comprises a chip substrate; at least one surface of the chip substrate is provided with a plurality of isolated single-stranded nucleic acid amplification clusters; single-stranded nucleic acid molecules in the single-stranded nucleic acid amplification cluster have identical nucleotide sequences, and nucleotide sequences of different single-stranded nucleic acid amplification clusters are different; the single-stranded nucleic acid amplification cluster has a known physical coordinate set relative to the spatial chip, and the single-stranded nucleic acid molecule comprises a barcode sequence with a known sequence and a first capture sequence and a second capture sequence respectively linked to the two ends of the barcode sequence; the first capture sequence is linked to a surface of the chip substrate, and the barcode sequences of each single-stranded nucleic acid amplification clusters are in one-to-one correspondence with the physical coordinate sets; the second single-stranded nucleotide library comprises a first base sequence, a second base sequence, and a capture part; the first base sequence is a known sequence, the second base sequence is a label sequence, and first base sequences of the nucleotides in the second single-stranded nucleotide library are identical, but the label sequences are different;
   the capture part is a transposase, and the first base sequence and the second base sequence are located in the transposase;
   contacting a cell or tissue sample of interest with the spatial chip and the second single-stranded nucleotide library to bond ATAC in the cell or tissue sample of interest on the spatial chip via the second single-stranded nucleotide library;
   cleaving the spatial chip, and collecting a cleavage product; and
   sequencing the cleavage product, and analyzing obtained sequencing data;
   optionally, when the cell or tissue sample of interest is a tissue sample, the tissue sample is a tissue section;
   optionally, the tissue section has a thickness of less than or equal to 50 µm;
   optionally, the step of contacting the cell or tissue sample of interest with the spatial chip and the second single-stranded nucleotide library comprises:
      contacting the cell or tissue sample of interest with the spatial chip, and then adding the second single-stranded nucleotide library;
   optionally, analyzing the obtained sequencing data comprises:
      on the basis of the sequencing data, acquiring positional information in the spatial chip of the barcode sequence in the cleavage product; and
      on the basis of the positional information, performing spatial restoration on the cleavage product to achieve ATAC sequencing analysis.
22. The use according to item 19, wherein the use is a method for spatial protein analysis comprising:
   providing the spatial chip and a second single-stranded nucleotide library, wherein the spatial chip comprises a chip substrate; at least one surface of the chip substrate is provided with a plurality of isolated single-stranded nucleic acid amplification clusters; single-stranded nucleic acid molecules in the single-stranded nucleic acid amplification cluster have identical nucleotide sequences, and nucleotide sequences of different single-stranded nucleic acid amplification clusters are different; the single-stranded nucleic acid amplification cluster has a known physical coordinate set relative to the spatial chip, and the single-stranded nucleic acid molecule comprises a barcode sequence with a known sequence and a first capture sequence and a second capture sequence respectively linked to the two ends of the barcode sequence; the first capture sequence is linked to a surface of the chip substrate; the second single-stranded nucleotide library comprises a first base sequence, a second base sequence and a capture part that are linked in sequence; the first base sequence is a known sequence complementary with the second capture sequence, the second base sequence is a label sequence, and first base sequences of the nucleotides in the second single-stranded nucleotide library are identical, but the label sequences are different;
   contacting a cell or tissue sample of interest with the spatial chip and the second single-stranded nucleotide library to bond a target having a binding capacity with the capture part in the sample of interest on the spatial chip via the second single-stranded nucleotide library;
   cleaving the spatial chip, and collecting a cleavage product; and
   sequencing the cleavage product, and analyzing obtained sequencing data;
   optionally, the capture part is a nucleic acid aptamer, a protein, an enzyme, or an antibody;
   optionally, the sample of interest is the cell or tissue sample of interest;
   optionally, when the cell or tissue sample of interest is a tissue sample, the tissue sample is a tissue section;
   optionally, the tissue section has a thickness of less than or equal to 50 µm;
   optionally, the step of contacting the cell or tissue sample of interest with the spatial chip and the second single-stranded nucleotide library comprises:
      contacting the cell or tissue sample of interest with the spatial chip, and then adding the second single-stranded nucleotide library;
      optionally, analyzing the obtained sequencing data comprises:
      on the basis of the sequencing data, acquiring positional information in the spatial chip of the barcode sequence in the cleavage product; and
      on the basis of the positional information, performing spatial restoration on the cleavage product to achieve spatial protein analysisAccording to the spatial chip and the method provided by the present application, barcode sequences in one-to-one correspondence with the positions of the nucleic acid clusters are provided on the surface. Therefore, when the spatial chip is used in spatial omics applications, in-situ capture of samples such as cells and tissues can be achieved by the nucleic acid molecules released from the spatial chip, and the spatial restoration can be achieved by the barcode sequences on the nucleic acid molecules to give spatial omics information.

### BRIEF DESCRIPTION OF THE DRAWINGS

To more clearly illustrate the technical schemes in the examples of the present application, the drawings required for use in the description of the examples or the prior art will be briefly described below. It is obvious that the drawings in the description below are only some examples of the present application, and other drawings can be derived from the drawings by those of ordinary skill in the art without creative efforts.
FIG. 1 illustrates a schematic of a chip substrate according to examples of the present application;
FIG. 2 illustrates a schematic of a spatial chip according to examples of the present application;
FIG. 3 illustrates a schematic of an array layout according to examples of the present application;
FIG. 4 illustrates a schematic of an array layout according to examples of the present application;
FIG. 5 illustrates a schematic of a spatial chip according to examples of the present application;
FIG. 6 illustrates a schematic of a spatial chip according to examples of the present application;
FIG. 7 illustrates a schematic of the composition of a single-stranded nucleic acid molecule and a second single-stranded nucleotide library according to examples of the present application;
FIG. 8 illustrates a structural schematic of a chip mother substrate according to examples of the present application;
FIG. 9 illustrates a schematic of preset chip regions on a chip mother substrate according to examples of the present application;
FIG. 10 illustrates the sequencing base distribution according to Example 1 of the present application;
FIG. 11 illustrates the spatial transcriptome bin50µm data statistics of the spatial omics chip according to Example 2 of the present application;
FIG. 12 illustrates the analysis results for spatial transcriptome clustered data of the spatial omics chip according to Example 2 of the present application;
FIG. 13 illustrates the fluorescence detection on the surface of the chip according to Example 3 of the present application;
FIG. 14 illustrates the nucleic acid fragment analysis according to Example 4 of the present application;
FIG. 15 illustrates the bin50µm data statistics in the Free RNA spatial capture method according to Example 5 of the present application; and
FIG. 16 illustrates the visual effect of bin50µm data restoration in the Free RNA spatial capture method according to Example 5 of the present application.

### DETAILED DESCRIPTION

In order to make the technical problems, technical schemes, and beneficial effects to be solved by the present application more apparent, the present application will be further described in detail below with reference to the examples. It will be appreciated that the specific examples described herein are intended to illustrate the present application only, rather than limit the present application.

In the present application, the terms used in the examples of the present application are for the purpose of describing particular examples only and are not intended to be limiting to the present application. The term "and/or" describes the associative relationship between associated objects, and refers to three possible relationships. For example, A and/or B may denote that: A is present alone, A and B are present simultaneously, and B is present alone. A and B may be singular or plural. The character "/" in "and/or" denotes that the former and latter associated objects are in an "and" relationship.

The term "at least one" means one or more, and "a plurality of" means two or more. The "at least one" or similar expressions thereof refer to any combination of these items, including any combination of the singular or plural items. For example, "at least one of a, b, or c" or "at least one of a, b, and c" may each refer to: a, b, c, a-b (i.e., a and b), a-c, b-c, or a-b-c, where a, b, and c may be a single item or a plurality of items, respectively. As used in the examples and the appended claims of the present application, the singular forms "a", "an", and "the" are intended to include the plural forms as well, unless the context clearly indicates otherwise.

The terms "first" and "second" are used for illustrative purposes only, are used for purposes distinguishing features such as substances, orientations, interfaces, messages, requests, and terminals from one another, and should not be construed as indicating or implying relative importance or implicitly indicating the number of the technical features indicated. For example, a first surface may also be referred to as a second surface, while a second surface may be referred to as a first surface, without departing from the scope of the examples of the present application. Therefore, features defined by "first" and "second" may explicitly or implicitly include one or more of the features.

In the description of the present application, the concentration of the related components mentioned herein may not only refer to the specific content of each component, but also refer to the proportional relationship of the content among components. Therefore, as long as the content of the related components is scaled up or down according to the examples of the specification in the present application, it is within the scope disclosed in the examples of the specification in the present application.

It will be appreciated that in the examples of the present application, the serial numbers of the processes described above do not imply an execution sequence, some or all of the steps may be executed in parallel or executed sequentially, and the execution sequence of each process should be determined by its function and inherent logic and should not constitute any limitation to the implementation process of the examples of the present application.

In the examples of the present application, the term "sequencing" may also be referred to as "nucleic acid sequencing" or "gene sequencing". That is, the three are used interchangeably to refer to the determination of the type and order of bases in a nucleic acid sequence, including the procedures of binding nucleotides (including nucleotide analogs) to a template and acquiring the corresponding signals emitted by the nucleotides (including nucleotide analogs). The "sequencing" includes sequencing by synthesis (SBS) and/or sequencing by ligation (SBL); includes DNA sequencing and/or RNA sequencing; includes long fragment sequencing and/or short fragment sequencing (the long fragment and short fragment are defined relatively; for example, nucleic acid molecules longer than 1 Kb, 2 Kb, 5 Kb or 10 Kb may be referred to as long fragments, and nucleic acid molecules shorter than 1 Kb or 800 bp may be referred to as short fragments); and includes double-end sequencing, single-end sequencing, paired-end sequencing, and/or the like, where the double-end sequencing or paired-end sequencing may refer to the reading of any two segments or portions of the same nucleic acid molecule that are not completely overlapping.

The term "nucleotide" refers to a molecule comprising a sugar, at least one phosphate group, and a nucleobase. The nucleotides include deoxyribonucleotides, modified deoxyribonucleotides, ribonucleotides, modified ribonucleotides, peptide nucleotides, modified peptide nucleotides, modified sugar-phosphate backbone nucleotides, and mixtures thereof. Examples of the nucleotide include adenosine monophosphate (AMP), adenosine diphosphate (ADP), adenosine triphosphate (ATP), thymidine monophosphate (TMP), thymidine diphosphate (TDP), thymidine triphosphate (TTP), cytidine monophosphate (CMP), cytidine diphosphate (CDP), cytidine triphosphate (CTP), guanosine monophosphate (GMP), guanosine diphosphate (GDP), guanosine triphosphate (GTP), uridine monophosphate (UMP), uridine diphosphate (UDP), uridine triphosphate (UTP), deoxyadenosine monophosphate (dAMP), deoxyadenosine diphosphate (dADP), deoxyadenosine triphosphate (dATP), deoxythymidine monophosphate (dTMP), deoxythymidine diphosphate (dTDP), deoxythymidine triphosphate (dTTP), deoxycytidine diphosphate (dCDP), deoxycytidine triphosphate (dCTP), deoxyguanosine monophosphate (dGMP), deoxyguanosine diphosphate (dGDP), deoxyguanosine triphosphate (dGTP), deoxyuridine monophosphate (dUMP), deoxyuridine diphosphate (dUDP), and deoxyuridine triphosphate (dUTP).

As used herein, the term "nucleotide" is also intended to encompass any nucleotide analogs that are those comprising a modified nucleobase, sugar, and/or phosphate moiety as compared to naturally occurring nucleotides. Exemplary modified nucleobases include inosine, xanthine, hypoxanthine, isocytosine, isoguanine, 2-aminopurine, 5-methylcytosine, 5-hydoxymethylcytosine, 2-aminoadenine, 6-methyladenine, 6-methylguanine, 2-propylguanine, 2-propyladenine, 2-thiouracil, 2-thiothymine, 2-thiocytosine, 15-halouracil, 15-halocytosine, 5-propynyluracil, 5-propynylcytosine, 6-azouracil, 6-azocytosine, 6-azothymine, 5-uracil, 4-thiouracil, 8-haloadenine or guanine, 8-aminoadenine or guanine, 8-thioladenine or guanine, 8-sulfanyladenine or guanine, 8-hydoxyadenine or guanine, 5-halouracil or cytosine, 7-methylguanine, 7-methyladenine, 8-azaguanine, 8-azaadenine, 7-deazaguanine, 7-deazaadenine, 3-deazaguanine, 3-deazaadenine, etc. As is known in the art, certain nucleotide analogs cannot be incorporated into polynucleotides, for example, nucleotide analogs such as adenosine 5'-phosphosulfate. The nucleotide may comprise any suitable number of phosphates, for example, three, four, five, six, or more than six phosphates.

The term "nucleic acid molecule" refers to a polymeric form of nucleotides of any length, and may comprise ribonucleotides, deoxyribonucleotides, analogs thereof, or mixtures thereof. The term may refer to a single-stranded polynucleotide or double-stranded polynucleotide. Nucleotides in a nucleic acid molecule may include naturally occurring nucleotides and functionally alternative analogs thereof. Examples of analogs can hybridize to nucleic acids in a sequence-specific manner, or can be used as templates for the replication of particular nucleotide sequences. Naturally occurring nucleotides generally have a main chain containing a phosphodiester bond. Analog structures may have alternative main chain linkages including any types known in the art. Naturally occurring nucleotides generally have deoxyribose (e.g., found in DNA) or ribose (e.g., found in RNA). Analog structures may have alternative sugar moieties including any types known in the art. Nucleotides may contain natural bases or non-natural bases. Bases in natural DNA may include one or more of adenine, thymine, cytosine, and/or guanine, and bases in natural RNA may include one or more of adenine, uracil, cytosine, and/or guanine. Any non-natural base or base analog may also be used in a nucleotide, such as a locked nucleic acid (LNA) and a bridged nucleic acid (BNA).

The "polynucleotide" refers to a molecule comprising a sequence of nucleotides that bind to each other. The polynucleotide is one non-limiting example of a polymer. Examples of the polynucleotide include deoxyribonucleic acid (DNA), ribonucleic acid (RNA), and analogs thereof. The polynucleotide may be a single-stranded sequence of nucleotides such as RNA or single-stranded DNA, or a double-stranded sequence of nucleotides such as double-stranded DNA, or may comprise a mixture of single-stranded and double-stranded sequences of nucleotides. The double-stranded DNA (dsDNA) includes genomic DNA, and PCR amplification products. The single-stranded DNA (ssDNA) can be converted to dsDNA, and *vice versa.* The polynucleotide may include non-naturally occurring DNA, such as enantiomeric DNA. The following are examples of the polynucleotide: gene or gene fragment (e.g., a probe, primer, expressed sequence tag (EST), or serial analysis of gene expression (SAGE) tag), genomic DNA, genomic DNA fragment, exon, intron, messenger RNA (mRNA), transfer RNA, ribosomal RNA, ribozyme, cDNA, recombinant polynucleotide, synthetic polynucleotide, branched polynucleotide, plasmid, vector, isolated DNA of any sequence, isolated RNA of any sequence, and nucleic acid probe, primer or amplified copies of any of the foregoing.

The term "barcode sequence" refers to a fragment of nucleotides in a nucleic acid molecule having a unique sequence on the spatial chip that can be used for identifying the nucleic acid, the characteristics of the nucleic acid, or an operation that has been performed on the nucleic acid. The barcode sequence may be a naturally occurring sequence or a synthetic sequence absent in nature. The barcode sequence in each nucleic acid molecule cluster is unique to the population of nucleic acid molecule clusters of the spatial chip.

The term "primer", also known as "probe", refers to an oligonucleotide or a nucleic acid molecule that can hybridize to a target sequence of interest. In the examples, the primer serves as a substrate onto which nucleotides may be polymerized by a polymerase. For example, the primer may be used as a starting point for DNA or RNA synthesis. For example, a sequencing primer can hybridize to a synthesized nucleic acid template strand so as to trigger the synthesis of a new strand complementary to the synthesized nucleic acid template strand. The primer may comprise any combination of nucleotides or analogs thereof. In some examples, the primer is a single-stranded oligonucleotide or polynucleotide.

The term "link" refers to the state of two objects engaging, fastening, adhering, attaching, or bonding to each other. For example, a nucleic acid molecule can be attached to a material such as a gel or solid support by a covalent or non-covalent bond. The covalent bond is characterized by a shared pair of electrons between atoms; the non-covalent bond is a chemical bond that does not involve the share of electron pairs and may include, for example, hydrogen bond, ionic bond, Van der Waals force, hydrophilic interaction, and hydrophobic interaction.

The term "cleavable site" refers to a position in a nucleic acid molecule susceptible to bond breakage. Such a position may be specific to a particular chemical, enzymatic, or physical process that results in bond breakage. For example, the position can be an abasic nucleotide or a nucleotide having a base that is readily removed to produce an abasic site. Examples of nucleotides prone to base removal include uracil and 8-oxo-guanine. The position may also be at or near a recognizable sequence of a restriction endonuclease, e.g., a nickase.

The term "cluster" refers to a population of nucleic acid molecules linked to the surface of the solid substrate to form a feature or site, where such nucleic acid molecules in the population of nucleic acid molecules typically are identical in sequence and can form a single clonal cluster by amplification or cloning. A non-monoclonal cluster mainly comprises an amplicon from a first nucleic acid, and may also have a lower level of contaminating amplicons from a second nucleic acid. For example, when using a cluster array in a detection application, an acceptable contamination level is one that does not affect the signal-to-noise ratio or resolution of the detection technique in an unacceptable manner. Thus, apparent clonality is generally associated with the particular use or application of the array prepared by the methods described herein.

The present disclosure will be described with the above interpretations.

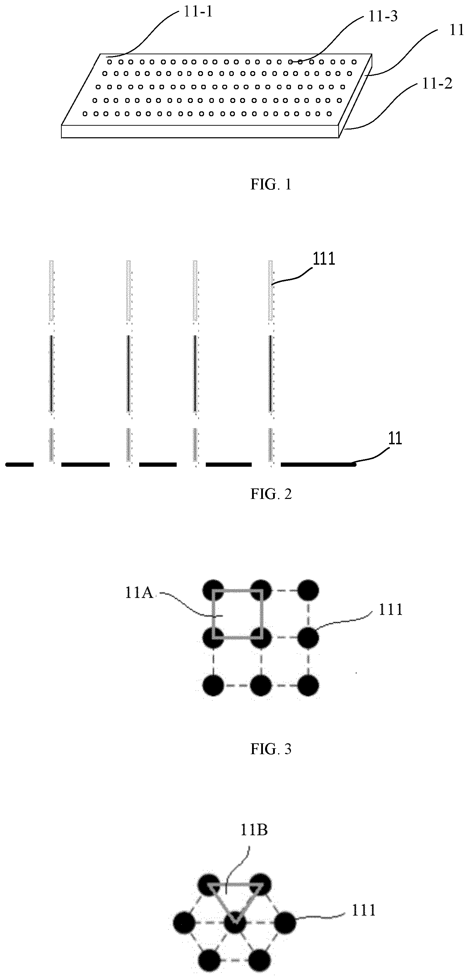

The examples of the present application, with reference to FIGs. 1-7, provide a spatial chip 10 comprising a chip substrate 11, and the chip substrate 11 is configured for carrying nucleic acid molecules and providing a reaction site for an identification based on nucleic acid molecules, a feature detection of nucleic acid molecules, and other operations performed on nucleic acid molecules.

The chip substrate 11 is provided with a solid substrate comprising a first surface 11-1 and a second surface 11-2 arranged opposite to the first surface 11-1, as shown in FIG. 1.

The material of the chip substrate 11 may be selected from quartz, glass, crystal, silicon wafer, silicon nitride, silicon dioxide, etc., and may also be plastic, ceramic, polyethylene terephthalate (PET), poly(methyl methacrylate) (PMMA), hydrogel, metal, or any other suitable material. The chip substrate 11 may have a flexibly set shape, or may have a special shape according to the material of the chip substrate 11. In one embodiment, the chip substrate 11 has a planar surface. In another embodiment, the chip substrate 11 may have a non-planar surface. Illustratively, the chip substrate 11 is a microbead or a magnetic bead, and at this point, the chip substrate 11 has a curved surface such as a spherical surface or a hemispherical surface. Certainly, in addition to the two above examples, the surface of the chip substrate 11 may be modified to form a modifier containing, for example, a dendrimer on the surface, and at this point, the surface may be an irregular surface with undulations or roughness.

When the chip substrate 11 has a planar surface, in some examples, the overall shape of the chip substrate 11 may be a sheet structure having a rectangular, square, rhombic, polygonal, circular, elliptical, or irregular shape. In some examples, the overall shape of the chip substrate 11 is configured into a rectangular sheet structure to fit most fixation devices for spatial chips and/or the structures for receiving and fixing the chip substrate 11 in most apparatuses. Moreover, in some certain embodiments, array chips in which the site information (including the barcode sequence to which the site is bonded at each site on the chip substrate 11 and the physical coordinate set of the barcode sequence) in each chip substrate 11 is known can be prepared in advance and in batch by cutting the sequencing region to form the chip substrate 11 after a general sequencing of the chip for sequencing, thereby achieving the preparation of spatial chips. In one implementation, on the basis of the rectangular sequencing region of the sequencing chip, the resultant chip substrate 11 may have a rectangular shape. In another implementation, the sequencing region of the sequencing chip can be cut to give a square chip substrate 11. It will be appreciated that the chip substrate 11 provided by the examples of the present application can be used as a nucleic acid sequencing carrier, and can also be used as a carrier for detecting nucleic acids for other purposes. Illustratively, the sources of the chip substrate 11 and the characteristics of the chip are not limited, and the chip substrate 11 includes, but is not limited to, sequencing chips (also referred to as flowcells) provided by various sequencing platforms such as Illumina, GeneMind, MGI, etc., sequencing microbeads provided by platforms such as ThermoFisher and Ultima, chip substrates identical or similar to gene chips provided by Affymatrix, etc.

In the examples of the present application, when the chip substrate 11 generally has a planar surface, micropores and/or nanopores(collectively referred to as micro/nanopores), or depressions, may be formed on at least one surface, specifically, the first surface, of the chip substrate 11; or microcolumns and/or nanocolumns(collectively referred to as micro/nanocolumns), or protrusions, may be formed on the surface , specifically, the first surface, of the chip substrate 11. In the examples of the present application, the micro-scale and /or nano-scale depressions and protrusions are collectively referred to as micro/nano structures 11-3, and the cross-sectional profiles of the micro/nano structures 11-3 such as micro/nanopores and micro/nanocolumns are not strictly limited, and may be circular, square, rectangular, rhombic, elliptical, polygonal (especially regular polygon), and irregular. The micro/nanopores are pores or depressions with a micron-scale or nano-scale diameter, and the micro/nanocolumns are columnar protrusions with a micron-scale or nano-scale maximum radial dimension of the cross-section. In some examples, the micro/nanopores or micro/nanocolumns are arrayed on the surface of the chip substrate 11; that is: the chip substrate 11 is provided with a plurality of micro/nano structures 11-3 arranged according to a preset rule.

In some certain embodiments, the minimum distance between adjacent micro/nano structures 11-3 is 10 nm to 100 µm. It will be appreciated that when the cross-sectional profile of the micro/nano structure 11-3 has a regular shape such as a circle, an ellipse, a square, a rectangle, other regular polygons, etc., the minimum distance between adjacent micro/nano structures 11-3 refers to the center-to-center distance between adjacent micro/nano structures 11-3; when the cross-sectional profile of the micro/nano structure 11-3 has an irregular shape, the minimum distance between adjacent micro/nano structures 11-3 refers to the shortest one among all connecting lines of any point on the circumference of one micro/nano structure 11-3 and any point on the circumference of an adjacent micro/nano structure 11-3. In some examples, the minimum distance between adjacent micro/nano structures 11-3 may be within a range of 50 nm to 10 µm, 100 nm to 1 µm, 200 nm to 800 nm, or any interval within a range of 10 nm to 100 µm. According to the micro/nano structures 11-3 of the examples of the present application, the minimum distances between the adjacent micro/nano structures 11-3 may be completely identical or different in the range described above. In some examples, the micro/nano structures 11-3 are arrayed on the first surface 11-1 of the chip substrate 11, and the minimum distances between any two adjacent micro/nano structures 11-3 are identical.

In some certain embodiments, the micro/nano structure 11-3 has a shape with a maximum size of 10 nm to 100 µm on the surface of the chip substrate 11. When the micro/nano structure 11-3 is a surface pattern formed on the first surface 11-1, the shape of the micro/nano structure 11-3 formed on the first surface 11-1 is a pattern shape, and the maximum size of the shape is the maximum distance between any two points on the circumference of the shape. When the micro/nano structure 11-3 is a geometric structure such as a protrusion or a depression, the shape of the micro/nano structure 11-3 formed on the first surface 11-1 is the shape of the micro/nano structure 11-3 acquired by taking the first surface 11-1 as a cross-section. At this point, the maximum size of the shape of the micro/nano structure 11-3 formed on the first surface 11-1 is the maximum distance between any two points on the circumference of the cross-section. In some examples, the shape of the micro/nano structure 11-3 formed on the first surface 11-1 may have a maximum size within a range of 50 nm to 10 µm, 100 nm to 1 µm, 200 nm to 800 nm, or any interval within a range of 10 nm to 100 µm. According to the micro/nano structures 11-3 of the examples of the present application, the maximum sizes of the shapes of the micro/nano structures 11-3 formed on the first surface 11-1 may be completely identical or different in the range described above. In some examples, the micro/nano structures 11-3 are arrayed on the first surface, and the shapes and the maximum sizes of the micro/nano structures 11-3 formed on the first surface 11-1 are identical.

In one embodiment, the chip substrate 11 is made of a material having a light transmittance of greater than or equal to 90%. As such, the reaction or operation on the surface of the chip substrate 11 can be detected by available sequencing methods based on optical signal detection. Theoretically, both surfaces of the chip substrate 11 can be used as the detection surface or monitoring surface for optical signals, so as to expand the application mode of the spatial chip.

In some examples, the chip substrate 11 has a thickness of 0.05 to 0.3 mm. Illustratively, the chip substrate 11 has a thickness of 0.05 mm, 0.10 mm, 0.15 mm, 0.20 mm, 0.25 mm, 0.30 mm, etc. The thickness of the chip substrate 11 within this range is beneficial to maintaining the structural integrity and the information integrity of the chip substrate 11 when the chip substrate 11 is separated from the chip mother substrate, and is advantageous for an optical system to image the optical signals generated on the surface of the chip substrate 11, so as to give optical images.

In the examples of the present application, as shown in FIG. 2, at least one surface of the chip substrate 11 is provided with a plurality of isolated single-stranded nucleic acid amplification clusters 111. The single-stranded nucleic acid amplification clusters 111 may be randomly distributed on the surface of the chip substrate 11, or may be distributed in an array on the surface of the chip substrate 11. In some embodiments, the single-stranded nucleic acid amplification cluster 111 is bonded to a modified surface of the chip substrate 11, including at least the first surface 11-1. In some other embodiments, the single-stranded nucleic acid amplification cluster 111 may be bonded to the surface of the micro/nanopores or micro/nanocolumns of the chip substrate 11. That is, the chip substrate 11 is a nanowell or nanopore chip, and the single-stranded nucleic acid amplification cluster 111 is immobilized in the nanowell or nanopore of the chip substrate 11.

In some certain embodiments, the single-stranded nucleic acid amplification cluster 111 is formed in the micro/nanopore structure of the chip substrate 11. In some examples, the depth of the micro/nanopore structure is 10 nm to 100 µm so as to meet the space required by sample binding or reaction; in some examples, the depth of the micro/nanopore structure may be 100 nm to 1 µm, or 200 nm to 800 nm. In some embodiments, the micro/nanopore structure is a circular pore structure, and in this embodiment, the diameter of the micro/nanopore structure is 10 nm to 100 µm so as to meet the space required by sample binding or reaction; in some examples, the diameter of the micro/nano pore structure may be 50 nm to 10 µm, 100 nm to 1 µm, or 200 nm to 800 nm.

In some certain embodiments, the distribution of the single-stranded nucleic acid amplification clusters 111 on the chip substrate 11 may be random. That is, the single-stranded nucleic acid amplification clusters 111 are randomly distributed on the chip substrate 11. As one implementation, the single-stranded nucleic acid amplification clusters 111 are arrayed on the chip substrate 11, such that the reaction signals generated by the detection reaction of the regularly arranged single-stranded nucleic acid amplification clusters 111 may have better recognizability. Illustratively, when the detection reaction on the surface of the single-stranded nucleic acid amplification clusters 111 generates optical signals, the regularly distributed optical signals are beneficial to data analysis or algorithm statistics and to acquiring corresponding detection parameters or detection results, or on the basis of the arrayed optical signals acquired at the single-stranded nucleic acid amplification clusters 111, the accuracy of the detection analysis results and the detection efficiency can be improved.

In some implementations, the single-stranded nucleic acid amplification clusters 111 may form regularly arranged array units 11A on the first surface of the chip substrate 11. In one embodiment, as shown in FIG. 3, the single-stranded nucleic acid amplification clusters 111 form array units 11A on the surface of the chip substrate 11. Each array unit 11A comprises four single-stranded nucleic acid amplification clusters 111, and the four single-stranded nucleic acid amplification clusters 111, when sequentially connected, form a rectangle. The array units 11A are regularly arranged on the chip substrate 11. The rectangles formed by connecting the four single-stranded nucleic acid amplification clusters 111 in the array units 11A have equal longer sides and equal shorter sides. That is, the rectangles formed by connecting the four single-stranded nucleic acid amplification clusters 111 in the array units 11A in the same manner are congruent rectangles. Furthermore, the rectangles formed by connecting the four single-stranded nucleic acid amplification clusters 111 in the adjacent array units 11A in the same manner share one longer side or one shorter side. In some examples, the rectangle may be a square, and at this point, any one of the single-stranded nucleic acid amplification clusters 111 other than those at the edges (such as the single-stranded nucleic acid amplification cluster 111 in the center of FIG. 2) may be simultaneously used as the vertex of the array units 11A.

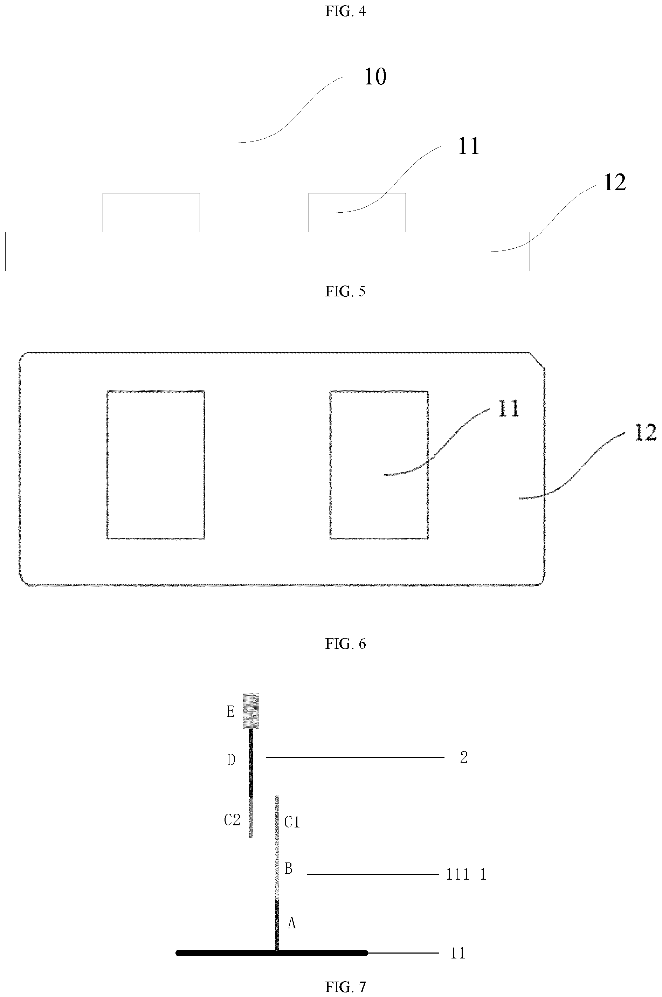

In one embodiment, as shown in FIG. 4, the chip substrate 11 is provided with several array units 11B. Each array unit 11B comprises three single-stranded nucleic acid amplification clusters 111, and the three single-stranded nucleic acid amplification clusters 111, when connected in pairs, form an isosceles triangle. The array units 11B are regularly arranged on the chip substrate 11. The isosceles triangles formed by connecting the three single-stranded nucleic acid amplification clusters 111 in the array units 11B have equal legs, equal bases, and equal vertex angles and base angles. That is, the isosceles triangles formed by connecting the three single-stranded nucleic acid amplification clusters 111 in the array units 11B in pairs are congruent triangles. Furthermore, the isosceles triangles formed by connecting the three single-stranded nucleic acid amplification clusters 111 in the adjacent array units 11B in pairs share one leg or one base. In some examples, the isosceles triangle may be an equilateral triangle. At this point, any one of the single-stranded nucleic acid amplification clusters 111 other than those at the edges (such as the single-stranded nucleic acid amplification cluster 111 in the center of FIG. 3) has six nearest single-stranded nucleic acid amplification clusters 111 nearest to and equidistant from the single-stranded nucleic acid amplification cluster 111. The six equidistant single-stranded nucleic acid amplification clusters 111 may be sequentially connected to form a regular hexagon.

The single-stranded nucleic acid amplification cluster 111 comprises a plurality of single-stranded nucleic acid molecules 111-1 with identical nucleotide sequences. In some certain embodiments, the number of the amplification clusters 111 on the chip substrate 11 is n, the number of nucleotides constituting the barcode sequence is m, and n and m satisfy: 4^{m} ≥ n. Thus, by arranging and combining the four nucleotides A, T or U, G, and C, 4^{m} different barcode sequences are obtained, and thereby, a barcode sequence having a unique sequence can be formed for each of the single-stranded nucleic acid amplification clusters 111 of the chip substrate 11. That is, the barcode sequences of the first single-stranded nucleotide libraries are different from each other.

In some examples, n is a natural number greater than or equal to 28; At this point, at least 4²⁸ barcodes with different sequences can be formed by 28 or more nucleotides, so as to meet the number requirement of the single-stranded nucleic acid amplification clusters 111 on the surface of the chip substrate 11 with a size of 1000 mm² or less.

In the examples of the present application, the density of amplification clusters can be set on the chip substrate 11 on the basis of the complexity of different reaction samples, so as to keep a better balance between the detection throughput and the accuracy of the detection results. In some certain embodiments, the area of the single-stranded nucleic acid amplification cluster 111 in the chip substrate 11 is 0.25-100 µm². Illustratively, the area of the single-stranded nucleic acid amplification cluster 111 in the chip substrate 11 may be 0.25 µm², 0.5 µm², 0.8 µm², 1.0 µm², 5.0 µm², 10.0 µm², 15.0 µm², 20.0 µm², 25.0 µm², 30.0 µm², 35.0 µm², 40.0 µm², 45.0 µm², 50.0 µm², 55.0 µm², 60.0 µm², 65.0 µm², 70.0 µm², 75.0 µm², 80.0 µm², 85.0 µm², 90.0 µm², 95.0 µm², 100 µm², etc.

In some certain embodiments, the distance between adjacent single-stranded nucleic acid amplification clusters 111 is 10 nm-100 µm. It will be appreciated that when the shape of the single-stranded nucleic acid amplification cluster 111 on the surface of the chip substrate 11 is a regular shape such as a circle, an ellipse, a square, a rectangle, other regular polygons, etc., the distance between adjacent single-stranded nucleic acid amplification clusters 111 refers to the center-to-center distance between adjacent single-stranded nucleic acid amplification clusters 111; when the shape of the single-stranded nucleic acid amplification cluster 111 on the surface of the chip substrate 11 is an irregular shape, the minimum distance between adjacent single-stranded nucleic acid amplification clusters 111 refers to the shortest one among all connecting lines of any point on the circumference of one single-stranded nucleic acid amplification cluster 111 and any point on the circumference of an adjacent single-stranded nucleic acid amplification cluster 111. In some examples, the distance between adjacent single-stranded nucleic acid amplification clusters 111 may be within a range of 50 nm to 10 µm, 100 nm to 1 µm, 200 nm to 800 nm, or any interval within a range of 10 nm to 100 µm. According to the single-stranded nucleic acid amplification clusters 111 of the examples of the present application, the distances between the adjacent single-stranded nucleic acid amplification clusters 111 may be completely identical or different in the range described above. In some examples, the single-stranded nucleic acid amplification clusters 111 are arrayed on a surface of the chip substrate 11, and the distances between any two adjacent single-stranded nucleic acid amplification clusters 111 are identical. Illustratively, the distance between adjacent single-stranded nucleic acid amplification clusters 111 may be 0.1 µm, 0.2 µm, 0.3 µm, 0.4 µm, 0.5 µm, 0.6 µm, 0.7 µm, 0.8 µm, 0.9 µm, 1.0 µm, 1.2 µm, 1.5 µm, 1.8 µm, 2.0 µm, 2.2 µm, 2.5 µm, 2.8 µm, 3.0 µm, 3.2 µm, 3.5 µm, 3.8 µm, 4.0 µm, 4.2 µm, 4.5 µm, 4.8 µm, 5.0 µm, 5.2 µm, 5.5 µm, 5.8 µm, 6.0 µm, 6.2 µm, 6.5 µm, 6.8 µm, 7.0 µm, 7.2 µm, 7.5 µm, 7.8 µm, 8.0 µm, 8.2 µm, 8.5 µm, 8.8 µm, 9.0 µm, 9.2 µm, 9.5 µm, 9.8 µm, 10.0 µm, etc.

In the examples of the present application, the density of the single-stranded nucleic acid amplification clusters 111 arranged on the chip substrate 11 is not strictly limited. In some certain embodiments, the position and density of the single-stranded nucleic acid amplification clusters 111 on the chip substrate 11 can be defined by performing a surface treatment on the chip substrate 11 to allow a plurality of separated positions that have undergone the surface treatment to bind to single-stranded nucleoside molecules and to prevent the surface of regions other than the plurality of separated positions from binding to single-stranded nucleoside molecules, thereby limiting the region and density of the distribution of the single-stranded nucleic acid amplification clusters 111. The form and the result of the surface treatment are not limited as long as the position and density of the single-stranded nucleic acid amplification clusters 111 on the chip substrate 11 can be defined. Illustratively, reactive groups capable of binding to the single-stranded nucleoside molecules may be formed at a part of the separated positions, or probes that are complementary to a partial sequence of the single-stranded nucleoside molecules may be linked to a part of the separated positions. In some examples, the density of the single-stranded nucleic acid amplification clusters 111 arranged on the chip substrate 11 is 5 to 800/mm². Illustratively, the density of the single-stranded nucleic acid amplification clusters 111 arranged on the chip substrate 11 may be 5 to 500/mm², 50 to 500/mm², 100 to 500/mm², 100 to 600/mm², 100 to 700/mm², 100 to 800/mm², 200 to 500/mm², 200 to 600/mm², 200 to 700/mm², 200 to 800/mm², 300 to 500/mm², 300 to 600/mm², 300 to 700/mm², 300 to 800/mm², 400 to 500/mm², 400 to 600/mm², 400 to 700/mm², 400 to 800/mm², 500 to 600/mm², 500 to 700/mm², 500 to 800/mm², 600 to 700/mm², 600 to 800/mm², 700 to 800/mm², etc.

In some certain embodiments, the copy number of the single-stranded nucleic acid molecules 111-1 of the single-stranded nucleic acid amplification cluster 111 in the chip substrate 11 may be 10 to 10⁵ polynucleotides. Illustratively, the copy number of the single-stranded nucleic acid molecules 111-1 of the single-stranded nucleic acid amplification cluster 111 in the chip substrate 11 may be 10, 20, 30, 40, 50, 60, 70, 80, 90, 100, 500, 800, 1000, 2000, 5000, 8000, 10000, 20000, 50000, 80000, 100000, etc. As such, the density of the single-stranded nucleic acid amplification clusters 111 of the chip substrate 11 may be up to 10 nmol/L or greater after release.

In the examples of the present application, nucleotide sequences of different single-stranded nucleic acid amplification clusters 111 are different. At least, referring to FIG. 7, the single-stranded nucleic acid molecule 111-1 comprises at least a barcode sequence B with a known sequence. That is, the barcode sequence B of each single-stranded nucleic acid amplification cluster 111 in the same chip substrate 11 is a unique sequence, and the position of each single-stranded nucleic acid amplification cluster 111 on the chip surface 11 can be defined via the barcode sequence B, such that the single-stranded nucleic acid amplification cluster 111 has a known physical coordinate set relative to the chip substrate 11. It will be appreciated that the positions of the single-stranded nucleic acid amplification clusters 111 in the chip substrate 11 are in one-to-one correspondence with the barcode sequences B of the single-stranded nucleic acid molecules 111-1. As such, after the signal acquired by the detection reaction is acquired, the barcode sequence B of the single-stranded nucleic acid amplification cluster 111 can be acquired on the basis of the physical coordinate set of the single-stranded nucleic acid amplification cluster 111, and other sequence regions of the polynucleotide linked the barcode sequence can be further acquired. On this basis, the analysis and result determination of the detection reaction are achieved. In the present application, physical coordinate refers to a position information which can be represented by (x, y). So, the physical coordinate of the single-stranded nucleic acid amplification clusters means the position information of the single-stranded nucleic acid amplification clusters on the chip substrate 11.

In some certain embodiments of the present application, the single-stranded nucleic acid molecule 111-1 further comprises a first capture sequence A. The first capture sequence A is located at one end of the barcode sequence B, and the barcode sequence B is linked to the chip substrate 11 via the first capture sequence A. That is, the first capture sequence A is linked to the chip substrate 11, and the barcode sequence B is located at the end of the first capture sequence A distal to the chip substrate 11. In some examples, the first capture sequence A is bonded to the chip substrate 11 by chemical bonding, physical adsorption, electrostatic adsorption, or Van der Waals force.

In one implementation, a compound is bonded to the chip substrate 11, and the first capture sequence A is linked to the chip substrate 11. The first capture sequence A is bonded to the chip substrate 11 by a linkage to the compound, in particular a reactive group of the compound. In some examples, a surface of the chip substrate 11 is provided with a first group, the compound is provided with a second group, the first group and the second group are a chemically reactive group set, and the compound is bonded to the chip substrate 11 via the group set. In some examples, the group set is selected from an amino-carboxylic acid set, an amino-ester set, an amino-halogen set, an amino-formyl set, an amino-epoxy group set, a sulfydryl-imino set, an azido-alkynyl set, an azido-alkenyl set, and a cycloalkenyl-tetrazinyl set. It will be appreciated that the two groups of the group set originate from the surface of the chip substrate 11 and the compound, respectively, and that either of the groups may originate from the surface of the chip substrate 11 while the other originates from the compound. Illustratively, the compound is selected from one or more of an amino compound, a carboxylate compound, succinimide or an analog thereof, a pentafluorophenol ester or an analog thereof, an aryl halide, maleimide or an analog thereof, an epoxy compound, a formyl-containing compound, an azido-containing compound, norbornene or an analog thereof, a DBCO or an analog thereof, and a tetrazine and an analog thereof.

In another implementation, the chip substrate 11 is provided with a first reactant, the first capture sequence A is provided with a second reactant, the first reactant and the second reactant are reactive, and the first capture sequence A is linked to the chip substrate 11 via the first reactant and the second reactant. In some examples, the first reactant is different from the second reactant. Illustratively, the first reactant and the second reactant are each independently selected from an antigen and an antibody, or the first reactant and the second reactant are each independently selected from biotin and streptavidin.

In some examples, a first spacer sequence region is arranged between the first capture sequence A and the barcode sequence B.

In some embodiments of the present application, the single-stranded nucleoside molecule 111-1 further comprises a second capture sequence C1, and the second capture sequence C1 is located at the end of the barcode sequence B distal to the first capture sequence A, that is, the second capture sequence C1 is linked to the barcode sequence B at the end distal to the first capture sequence A. At this point, the single-stranded nucleoside molecule 111-1 comprises, in sequence, the first capture sequence A, the barcode sequence B, and the second capture sequence C1. The second capture sequence C1 is used for capturing a sample of interest, and the sequence length and arrangement of the second capture sequence C1 can be determined according to the type and nature of the sample of interest.

In some examples, a second spacer sequence region is arranged between the second capture sequence C1 and the barcode sequence B.

In one example, the single-stranded nucleoside molecule 111-1 comprises the first capture sequence A and the barcode sequence B that are linked in sequence. In one example, the single-stranded nucleoside molecule 111-1 comprises the first capture sequence A, the first spacer sequence region, and the barcode sequence B that are linked in sequence. In one example, the single-stranded nucleoside molecule 111-1 comprises the first capture sequence A, the barcode sequence B, and the second capture sequence C1 that are linked in sequence. In one example, the single-stranded nucleoside molecule 111-1 comprises the first capture sequence A, the first spacer sequence region, the barcode sequence B, the second spacer sequence region, and the second capture sequence C1 that are linked in sequence. On the basis of the above examples, as one implementation, a PolyN sequence may be further arranged at the end of the barcode sequence B distal to the first capture sequence A or between the second capture sequence C1 and the barcode sequence B, where N is selected from one of A or a derivative thereof, T (or U) or a derivative thereof, G or a derivative thereof, and C or a derivative thereof. Illustratively, a PolyA sequence may be further arranged at the end of the barcode sequence B distal to the first capture sequence A or between the second capture sequence C1 and the barcode sequence B.

In some implementations, a cleavable site is arranged in the first capture sequence A and the second capture sequence C1. As such, after the spatial capture using the spatial chip, the single-stranded nucleic acid molecule 111-1 and the target captured by the same can be released by cleaving the cleavable site of the first capture sequence A. As such, in some implementations, the chip substrate 11 is a substrate capable of achieving the release of single-stranded nucleic acid molecules in a cleaving condition. The second capture sequence C1 can be amplified to form an amplification cluster on the surface of the chip substrate 11, and then the obtained double-stranded nucleic acid molecules are cleaved to achieve linearization, so as to give the single-stranded nucleic acid molecules 111-1.

In some examples, the cleavable site may be a photocleavable site, a chemically cleavable site, or an enzyme-cleavable site, or may be any two or three of the above three cleavable sites. It will be appreciated that when the cleavable site is an enzyme-cleavable site, the barcode sequence does not comprise a sequence identical to the cleavable site, so as to avoid the cleavage of the barcode sequence.

In some implementations, the spatial chip comprises two or more chip substrates 11, and the chip substrates 11 are immobilized on a surface of a solid carrier in a preset arrangement. Illustratively, as shown in FIG. 5, the spatial chip 10 comprises two or more chip substrates 11, and the two chip substrates 11 are immobilized on a solid carrier 12.

In the examples of the present application, each of the single-stranded nucleic acid amplification clusters 111 in the chip substrate 11 has a known physical coordinate set. When the spatial chip has two or more chip substrates 11, each of the chip substrates 11 has known positional information with respect to the spatial chip, such that each of the single-stranded nucleic acid amplification clusters 111 on each of the chip substrates 11 has known positional information with respect to the spatial chip.

In some certain embodiments, the chip substrate 11 is provided with one or more markers. By providing markers on the chip substrate 11, the arrangement direction of the chip substrate 11 on the surface of the solid carrier 12 and the site information of each of the single-stranded nucleic acid amplification clusters 111 in the chip substrate 11 can be acquired, the site information including the physical coordinate sets of the single-stranded nucleic acid amplification clusters 111 and the barcode sequence of each of the single-stranded nucleic acid amplification cluster 111. As such, when the spatial chip is adopted for spatial sample analysis, according to the barcode sequences, the spatial information of the bonded sample in the original sample can be determined via the physical coordinate sets corresponding to the barcode sequences.

In the examples of the present application, the marking mode of the marker is not limited strictly, and in some certain embodiments, one of a letter, a number and a graphic, or a combination thereof may be used. Illustratively, a marker of a letter alone, a marker of a number alone, a marker of a graphic alone, a marker comprising a letter and a number, a marker comprising a letter and a graphic, or a marker comprising a number and a graphic, or a marker comprising a letter, a number and a graphic may be used.

In some certain embodiments, the marker includes a directional marker and a regional marker. The directional marker is configured for indicating the orientation of the chip substrate 11 bonded to the surface of the solid carrier, such that the site information of each of the single-stranded nucleic acid amplification clusters 111 can be accurately determined in the subsequent detection. In one example, the directional marker may be an arrow marker or other markers having directivity to indicate the direction of the chip substrate 11.

In the examples of the present application, one chip substrate 11 may be provided with one or more directional markers. The positions for arranging the directional markers on the chip substrate 11 may be outside the region of the single-stranded nucleic acid amplification clusters 111 on the chip substrate 11, i.e., the edge region of the chip substrate 11. For example, when the chip substrate 11 is a rectangular chip, the directional marker may be arranged in regions where one or more right angles of the chip substrate 11 are located, so as to quickly distinguish the direction of the chip substrate 11.

In some examples, each of the chip substrates 11 may comprise one or more regional markers. When one regional marker is arranged on the chip substrate 11 of one chip substrate 11, for the detection reaction performed in each reaction region, the regional marker is used as a reference for position calibration to determine the site information (including the barcode sequence and the physical coordinate set of each of the single-stranded nucleic acid amplification clusters 111) of the reaction region, and then the signal or result of the corresponding detection reaction is acquired and analyzed.

As such, in some examples, the chip substrate 11 is provided with a plurality of regional markers. By arranging the plurality of regional markers, different regions on the surface of the chip substrate 11 can be separately marked, such that when the surface of the chip substrate 11 is subjected to a detection reaction, the relative or absolute position of the reaction region in the chip substrate 11 can be quickly learned via the regional markers, thus acquiring the corresponding site information. When a detection reaction is performed using the chip substrate 11, this method, on the basis of the current regional marker, can quickly achieve region calibration, quickly determine the site information (including the physical coordinate set of the single-stranded nucleic acid amplification cluster 111 and the barcode sequence corresponding to each positional information) of the region, and acquire a signal or a result of the corresponding detection reaction, thereby avoiding repeated calibration when using one marker as a reference for each reaction.

In the examples of the present application, when the chip substrate 11 is provided with the plurality of regional markers, the plurality of regional markers may be achieved by using different marker patterns, such as: different letters/numbers (including different letters and/or cases, such as A, B, C, D, a, b, c, d, etc.), different patterns, such as circles, triangles, rectangles, squares, ellipses, other polygons, etc., or different letters and patterns, etc. Certainly, the marker patterns may be distinguished by the arrangement at different arrangement angles relative to one direction of the chip substrate 11 (such as the orientation of the vertex angle of a non-equilateral triangle, etc.), or may be distinguished by using different numbers of marker patterns (such as a square or a triangle or a circle, two squares or triangles or circles regularly arranged in a certain arrangement rule, etc.).

In some certain embodiments, the plurality of regional markers are arranged on the surface where the single-stranded nucleic acid amplification cluster 111 is located and close to the circumference of the chip substrate 11. In some examples, the chip substrate 11 has a polygonal shape such as a rectangle, and the plurality of regional markers are uniformly distributed in a direction parallel to each side of the polygon. In one example, for the convenience of quick recognition, the types of the regional markers are different for markers arranged on different sides. For example, the four sides of a rectangle are respectively marked with circles, triangles, squares, and rectangles, and certainly, different patterns or different numbers of the same pattern may be used for marking. In another example, the chip substrate 11 is circular or elliptical, and the regional markers are uniformly distributed along a track close to the circumference. By uniformly dispersing the plurality of regional markers, the speed of positioning the detection region is increased, thereby improving the efficiency of the detection reaction and analysis of the spatial chip substrate 11.

In some certain embodiments, the marker further includes a chip serial number marker for marking the arrangement order of a plurality of chip substrates 11 on the solid carrier 12.

In some examples, the marker may further include a chip source marker for recording the region of the chip substrate 11 in the chip mother substrate before preparation, and the site information in the chip substrate 11 is further determined in conjunction with the source information of the chip substrate 11. By arranging source marker on the chip substrate 11, i.e., recording the positional information of the single-stranded nucleic acid amplification clusters 111 contained in the chip substrate 11 in the chip mother substrate, a unique barcode sequence corresponding to each of the single-stranded nucleic acid amplification clusters 111 contained in the chip substrate 11 is determined. In some examples of the present application, the chip source marker is a combination of a number and a letter. For example, in 3B, "3" denotes a first division on the mother chip, and "B" denotes a second division in the first division. Illustratively, when the mother chip is provided with four sequencing regions such as four sequencing channels, and three chip substrates 11 are sequentially separated in a certain direction in each sequencing region, 3B may denote the second chip substrate 11 separated in a preset direction from the third sequencing region of the large source chip.

In the examples of the present application, the solid carrier 12 is provided with a chip label; the chip label is a computer-storable information label, and the chip label is a QR code or a barcode or other labels or instructions that can be associated with computer-storable information for recording the physical coordinate sets of the amplification clusters 111 on each of the chip substrates 11 and the sequence of the barcode sequence region at each of the physical coordinate sets. By scanning the QR code or the barcode, the positions of the sites included in the chip substrate 11 and the unique barcode sequence corresponding to each position can be quickly acquired. In some examples, the chip label is arranged on the second surface 11-2 of the chip substrate 11. In some examples, the chip label is arranged on the surface of the solid carrier 12. Illustratively, the chip label may be, but is not limited to, a QR code or a barcode.

In the examples of the present application, a certain space is arranged between the edge of the chip substrate 11 and the solid carrier 12, so as to avoid abrasion of or damage to the edge of the chip substrate 11 during the processes of preparation, storage and transportation and thus the loss of site data. In some certain embodiments, the distance between the edge of the chip substrate 11 and the edge of the solid carrier 12 is greater than or equal to 1 mm. Illustratively, the distance between the edge of the chip substrate 11 and the edge of the solid carrier 12 is greater than or equal to 1 mm, 2 mm, 3 mm, 4 mm, 5 mm, 6 mm, etc., without limitation. In some examples, the distance between the edges of adjacent chip substrates 11 and solid carrier 12 is one quarter or more of the length of the chip substrate 11 in the direction of the distance.

In the examples of the present application, one chip substrate 11 may be arranged on one solid carrier 12, or two or more chip substrates 11 may be arranged simultaneously. The number and density of the chip substrates 11 on the solid carrier 12 can be determined in accordance with actual requirements. In some examples, the distance between adjacent chip substrates 11 is greater than or equal to 1 mm. Setting an appropriate distance between the adjacent chip substrates 11 can avoid cross-interference when conducting the detection reactions and thus detection crosstalk when performing signal acquisition or detection on the chip substrates 11 after the reaction.

It will be appreciated that when two or more chip substrates 11 are simultaneously arranged on one solid carrier 12, a plurality of chip substrates 11 may be arrayed on the surface of the solid carrier 12. In some examples, each of the chip substrates 11 has known positional information relative to the solid carrier 12. The positional information is acquired from the chip serial number marker of the chip substrate 11.

In some certain embodiments, a glue layer is arranged between the chip substrate 11 and the solid carrier 12, and the chip substrate 11 is fixed by the adhesive action of the glue layer. In some examples, the glue layer may cover the entire chip substrate 11 to completely adhere the chip substrate 11 to the surface of the solid carrier 12. In some other examples, a glue layer can be arranged on a part of the area of the chip substrate 11 to partially adhere the chip substrate 11 to the surface of the solid carrier 12. It will be appreciated that when using the same adhesive material and adhesion mode, a larger adhered area may result in a firmer bonding between the chip substrate 11 and the solid carrier 12. When the glue layer is arranged on a part of the area of the chip substrate 11, the area where the glue layer is arranged may be an edge area, or may be several bonding regions uniformly or non-uniformly arranged on the glue layer.

In some examples, the glue layer is made of a material having a light transmittance of greater than or equal to 90%, such as an optical adhesive, etc. As such, the obtained chip substrate 11 can be used for optical detection without being affected by the light transmittance of the glue layer. Illustratively, the glue layer is at least one selected from a transparent double-sided adhesive, an epoxy adhesive such as an epoxy resin, and an acrylate adhesive such as an acrylic resin.

The solid carrier 12 provided in the examples of the present application may be implemented in various ways, for example, by fixing the chip substrate 11 having complete functional information on the surface of the solid carrier 12, or by processing the chip substrate 11 bonded to the solid carrier 12 to form a micro/nano structure 11-3 having a physical coordinate set on the chip substrate 11 and a single-stranded nucleic acid molecule cluster having a plurality of identical single-stranded nucleoside molecules at the position of each micro/nano structure 11-3, where each of the single-stranded nucleic acid molecule clusters has a unique barcode sequence. In some embodiments, the solid carrier 12 is a solid support for carrying the chip substrate 11 and having a generally flat surface to achieve a flat distribution of the chip substrates 11 on the surface. The material of the solid carrier 12 is not strictly limited, and may be an inorganic material such as silicon dioxide, silicon nitride, silicon, quartz, glass, ceramic, etc., or an organic material such as a polymer substrate polyethylene terephthalate (PET), poly(methyl methacrylate) (PMMA), etc., that is generally used as a substrate or a carrier.

In some embodiments, the solid carrier 12 is selected from a solid carrier having a light transmittance of greater than or equal to 90%, such as transparent glass. As such, when the obtained spatial chip 10 is used for optical detection, the interference of the solid carrier with the detection signal can be avoided.

In some embodiments, the solid carrier 12 has a thickness of 0.5 to 1.5 mm. Illustratively, the thicknesses of the solid carrier 12 may be 0.5 ± 0.05 mm, 0.6 ± 0.05 mm, 0.7 ± 0.05 mm, 0.8 ± 0.05 mm, 0.9 ± 0.05 mm, 1.0 ± 0.05 mm, 1.1 ± 0.05 mm, 1.2 ± 0.05 mm, 1.3 ± 0.05 mm, 1.4 ± 0.05 mm, 1.5 ± 0.05 mm, etc. A thickness of the solid carrier 12 in this range is advantageous to the fixation of the spatial chip in a holder, and also to optical focusing and optical imaging when the spatial chip 10 is subjected to optical signal acquisition.

The shape of the solid carrier 12 is not limited strictly, and in one embodiment, the shape of the solid carrier 12 is a rectangular structure, and at least one of the four right angles of the rectangular structure is provided as a chamfer.

The chamfer in the examples of the present application may be, but is not limited to, a C-chamfer and/or an R-chamfer. The C-chamfer is sometimes referred to as a slope chamfer; the R-chamfer is also sometimes referred to as a round or a rounded corner. As shown in FIG. 6, two adjacent sides of the solid carrier 12 are connected by a line segment instead of a right angle, thereby forming the C-chamfer. It will be appreciated that the number of line segments forming the chamfer may be one, or may be two or more. When the C-chamfer is formed by a number of line segments, the line segments are sequentially connected to form a regular or irregular line, and the two ends of the regular or irregular line are respectively connected with the endpoints of the two adjacent edges of the rectangular substrate at the chamfer. The R-chamfer adopts an arc to replace the original right angle for connecting the endpoints of the two adjacent edges of the rectangular substrate. It will be appreciated that the number of the arcs forming the R-chamfer may be one, or may be two or more. When the R-chamfer is formed by a number of arcs, the arcs are sequentially connected to form a regular or irregular curve, and the two ends of the regular or irregular curve are respectively connected with the endpoints of the two adjacent edges of the rectangular substrate at the chamfer.

In some examples, as shown in FIG. 6, three of the right angles of the solid carrier 12 are provided as R-chamfers, and one of the right angles is provided as a C-chamfer. As such, the recognizability of the orientation and positioning of the solid carrier 12 can be enhanced.

The method for preparing the spatial chip provided by the examples of the present application is not strictly limited. As one implementation, the spatial chip is prepared by the following method.

The examples of the present application provide a method for preparing a spatial chip, comprising: S10, synthesizing a first single-stranded nucleotide library with a barcode sequence.

In the examples of the present application, the first single-stranded nucleotide library is a single-stranded nucleotide library comprising a specific barcode sequence, and the first single-stranded nucleotide library may be a polynucleotide sequence having a sequence identical to the single-stranded nucleic acid molecule described above, or a polynucleotide sequence complementary to the single-stranded nucleic acid molecule described above.

In the examples of the present application, nucleotide sequences of different first single-stranded nucleotide libraries are different. At least, the first single-stranded nucleotide library comprises at least a barcode sequence with a known sequence, and the barcode sequences of the different first single-stranded nucleotide libraries are unique sequences.

**In** some certain embodiments of the present application, the first single-stranded nucleotide library further comprises a first capture sequence, and the first capture sequence is located at one end of the barcode sequence. The first capture sequence is used for immobilizing the first single-stranded nucleotide library on the surface of the solid substrate or linking a capture sequence to capture the sample of interest via the capture sequence. In some examples, a first spacer sequence region is arranged between the first capture sequence and the barcode sequence.

**In** some embodiments of the present application, the first single-stranded nucleotide library further comprises a second capture sequence, and the second capture sequence is located at the end of the barcode sequence distal to the first capture sequence, that is, the second capture sequence is linked to the barcode sequence at the end distal to the first capture sequence. At this point, the single-stranded nucleoside molecule comprises, in sequence, the first capture sequence, the barcode sequence, and the second capture sequence. The second capture sequence is used for immobilizing the first single-stranded nucleotide library on the surface of the solid substrate or linking a capture sequence to capture the sample of interest via the capture sequence, and the sequence length and arrangement of the second capture sequence can be determined according to the type and nature of the sample of interest. In some examples, a second spacer sequence region is arranged between the second capture sequence and the barcode sequence.

In one example, the first single-stranded nucleotide library comprises the first capture sequence, the barcode sequence, and the second capture sequence that are linked in sequence. In one example, the first single-stranded nucleotide library comprises the first capture sequence, the first spacer sequence region, the barcode sequence, the second spacer region, and the second capture sequence that are linked in sequence. On the basis of the above examples, as one implementation, a PolyN sequence may be further arranged at the end of the barcode sequence distal to the first capture sequence or between the second capture sequence and the barcode sequence, where N is selected from one of A or a derivative thereof, T (U) or a derivative thereof, G or a derivative thereof, and C or a derivative thereof. Illustratively, a Poly A sequence may be further arranged at the end of the barcode sequence distal to the first capture sequence or between the second capture sequence and the barcode sequence.

In some implementations, a cleavable site is arranged in the first capture sequence and the second capture sequence. As such, through the cleavable site of the first capture sequence, after the spatial capture using the spatial chip, the single-stranded nucleic acid molecule and the target captured by the same can be released by cleaving the cleavable site of the first capture sequence. As such, in some implementations, the spatial chip is a substrate capable of achieving the release of single-stranded nucleic acid molecules in a cleaving condition. The second capture sequence can be amplified to form an amplification cluster on the surface of the solid substrate, and then the obtained double-stranded nucleic acid molecules are cleaved to achieve linearization, so as to give the single-stranded nucleic acid molecules.

In some examples, the cleavable site may be a photocleavable site, a chemically cleavable site, or an enzyme-cleavable site, or may be any two or three of the above three cleavable sites. It will be appreciated that when the cleavable site is an enzyme-cleavable site, the barcode sequence does not comprise a sequence identical to the cleavable site, so as to avoid the cleavage of the barcode sequence.

S20, immobilizing the first single-stranded nucleotide library on a surface of a solid substrate, and amplifying the first single-stranded nucleotide library to give an amplification cluster.

In the examples of the present application, the material of the solid substrate may be selected from quartz, glass, crystal, silicon wafer, silicon nitride, silicon dioxide, etc., and may also be plastic, ceramic, polyethylene terephthalate (PET), poly(methyl methacrylate) (PMMA), hydrogel, metal, or any other suitable material. The solid substrate may have a flexibly set shape on the basis of the requirement for the shape by the spatial chip, or may have a special shape according to the material of the solid substrate. In one embodiment, the solid substrate has a planar surface. In another embodiment, the solid substrate may have a non-planar surface. Illustratively, the solid substrate is a microbead or a magnetic bead, and at this point, the solid substrate has a curved surface such as a spherical surface or a hemispherical surface. Certainly, in addition to the two above examples, the surface of the solid substrate may be modified to form a modifier containing, for example, a dendrimer on the surface, and at this point, the surface may be an irregular surface with undulations or roughness.

In the examples of the present application, when the solid substrate generally has a planar surface, micro/nanopores, or depressions, may be formed on at least one surface of the solid substrate; or micro/nanocolumns, or protrusions, may be formed on the surface of the solid substrate. In the examples of the present application, the micro/nano-scale depressions and protrusions are collectively referred to as micro/nano structures, and the cross-sectional profiles of the micro/nano structures such as micro/nanopores and nanocolumns are not strictly limited, and may be circular, square, rectangular, rhombic, elliptical, polygonal (especially regular polygon), and irregular. The micro/nanopores are pores or depressions with a micron-scale or nano-scale diameter, and the micro/nanocolumns are columnar protrusions with a micron-scale or nano-scale maximum radial dimension of the cross-section. In some examples, the micro/nanopores or micro/nanocolumns are arrayed on the surface of the solid substrate. Illustratively, the surface of the solid substrate is provided with arrayed nanowells or nanopores, and the first single-stranded nucleotide libraries are immobilized in the nanowells or the nanopores, that is, the solid substrate is provided with a plurality of micro/nano structures arranged according to a preset rule.

In some examples, for the selection and characterization of the solid substrate, reference can be made to those of the chip substrate described above, which will not be recited here for brevity.

In some examples, in the step of immobilizing the first single-stranded nucleotide library on the surface of the solid substrate, the first capture sequence is bonded to the surface of the solid substrate. That is the first capture sequence is linked to the solid substrate, and the barcode sequence is located at the end of the first capture sequence distal to the solid substrate. In some examples, the first capture sequence is bonded to the surface of the solid substrate by chemical bonding, physical adsorption, electrostatic adsorption, or Van der Waals force.

In one implementation, a compound is bonded to the surface of the solid substrate, and the first capture sequence is linked to the surface of the solid substrate. The first capture sequence is bonded to the surface of the solid substrate by a linkage to the compound, in particular a reactive group of the compound. In some examples, the surface of the solid substrate is provided with a first group, the compound is provided with a second group, the first group and the second group are a chemically reactive group set, and the compound is bonded to the surface of the solid substrate via the group set. In some examples, the group set is selected from an amino-carboxylic acid set, an amino-ester set, an amino-halogen set, an amino-formyl set, an amino-epoxy group set, a sulfydryl-imino set, an azido-alkynyl set, an azido-alkenyl set, and a cycloalkenyl-tetrazinyl set. It will be appreciated that the two groups of the group set originate from the surface of the surface of the solid substrate and the compound, respectively, and that either of the groups may originate from the surface of the solid substrate while the other originates from the compound. Illustratively, the compound is selected from one or more of an amino compound, a carboxylate compound, succinimide or an analog thereof, a pentafluorophenol ester or an analog thereof, an aryl halide, maleimide or an analog thereof, an epoxy compound, a formyl-containing compound, an azido-containing compound, norbornene or an analog thereof, a DBCO or an analog thereof, and a tetrazine and an analog thereof.

In another implementation, the surface of the solid substrate is provided with a first reactant, the first capture sequence is provided with a second reactant, the first reactant and the second reactant are reactive, and the first capture sequence is linked to the solid substrate via the first reactant and the second reactant. In some examples, the first reactant is different from the second reactant. Illustratively, the first reactant and the second reactant are each independently selected from an antigen and an antibody, or the first reactant and the second reactant are each independently selected from biotin and streptavidin.

In the examples of the present application, the first single-stranded nucleotide library is amplified to give an amplification cluster. The method for amplifying the first single-stranded nucleotide library is not strictly limited in the examples of the present application, and may be a bridge amplification, or other methods that can achieve the amplification on a solid surface.

S30, linearizing the amplification cluster to give a single-stranded nucleic acid amplification cluster.

In the examples of the present application, the double-stranded amplification cluster is linearized; one strand is removed, while the other strand is retained, so as to give a single-stranded nucleic acid amplification cluster. In some examples, the step of linearizing the amplification cluster comprises: adding a restriction endonuclease, and digesting the amplification cluster at an enzyme-cleavable site.

In the examples of the present application, the single-stranded nucleic acid amplification cluster comprises a plurality of single-stranded nucleic acid molecules with identical nucleotide sequences. In some certain embodiments, the number of the amplification clusters on the surface of the solid substrate is n, the number of nucleotides constituting the barcode is m, and n and m satisfy: 4^{m} ≥ n. Thus, by arranging and combining the four nucleotides A, T or U, G, and C, 4^{m} different barcode sequences are obtained, and thereby, a barcode sequence having a unique sequence can be formed for each of the single-stranded nucleic acid amplification clusters of the surface of the solid substrate.

In some examples, n is a natural number greater than or equal to 28; At this point, at least 4²⁸ barcodes with different sequences can be formed by 28 or more nucleotides, so as to meet the number requirement of the single-stranded nucleic acid amplification clusters on the surface of the solid substrate with a size of 1000 mm² or less.

In the examples of the present application, the density of amplification clusters can be set on the surface of the solid substrate on the basis of the complexity of different reaction samples, so as to keep a better balance between the detection throughput and the accuracy of the detection results. In some certain embodiments, the area of the single-stranded nucleic acid amplification cluster in the surface of the solid substrate is 0.25-100 µm². Illustratively, the area of the single-stranded nucleic acid amplification cluster in the surface of the solid substrate may be 0.25 µm², 0.5 µm², 0.8 µm², 1.0 µm², 5.0 µm², 10.0 µm², 15.0 µm², 20.0 µm², 25.0 µm², 30.0 µm², 35.0 µm², 40.0 µm², 45.0 µm², 50.0 µm², 55.0 µm², 60.0 µm², 65.0 µm², 70.0 µm², 75.0 µm², 80.0 µm², 85.0 µm², 90.0 µm², 95.0 µm², 100 µm², etc.

In some certain embodiments, the distance between adjacent single-stranded nucleic acid amplification clusters is 10 nm-100 µm. It will be appreciated that when the single-stranded nucleic acid amplification cluster has a regular shape such as a circle, an ellipse, a square, a rectangle, other regular polygons, etc., the distance between adjacent single-stranded nucleic acid amplification clusters refers to the center-to-center distance between adjacent single-stranded nucleic acid amplification clusters; when the single-stranded nucleic acid amplification cluster has an irregular shape, the minimum distance between adjacent single-stranded nucleic acid amplification clusters refers to the shortest one among all connecting lines of any point on the circumference of one single-stranded nucleic acid amplification cluster and any point on the circumference of an adjacent single-stranded nucleic acid amplification cluster. In some examples, the distance between adjacent single-stranded nucleic acid amplification clusters may be within a range of 50 nm to 10 µm, 100 nm to 1 µm, 200 nm to 800 nm, or any interval within a range of 10 nm to 100 µm. According to the single-stranded nucleic acid amplification clusters of the examples of the present application, the distances between the adjacent single-stranded nucleic acid amplification clusters may be completely identical or different in the range described above. In some examples, the single-stranded nucleic acid amplification clusters are arrayed on the surface of the solid substrate, and the distances between any two adjacent single-stranded nucleic acid amplification clusters are identical. Illustratively, the distance between adjacent single-stranded nucleic acid amplification clusters may be 0.1 µm, 0.2 µm, 0.3 µm, 0.4 µm, 0.5 µm, 0.6 µm, 0.7 µm, 0.8 µm, 0.9 µm, 1.0 µm, 1.2 µm, 1.5 µm, 1.8 µm, 2.0 µm, 2.2 µm, 2.5 µm, 2.8 µm, 3.0 µm, 3.2 µm, 3.5 µm, 3.8 µm, 4.0 µm, 4.2 µm, 4.5 µm, 4.8 µm, 5.0 µm, 5.2 µm, 5.5 µm, 5.8 µm, 6.0 µm, 6.2 µm, 6.5 µm, 6.8 µm, 7.0 µm, 7.2 µm, 7.5 µm, 7.8 µm, 8.0 µm, 8.2 µm, 8.5 µm, 8.8 µm, 9.0 µm, 9.2 µm, 9.5 µm, 9.8 µm, 10.0 µm, etc.

In the examples of the present application, the density of the single-stranded nucleic acid amplification clusters arranged on the surface of the solid substrate is not strictly limited. In some certain embodiments, the position and density of the single-stranded nucleic acid amplification clusters on the surface of the solid substrate can be defined by performing a surface treatment on the surface of the solid substrate to allow a plurality of separated positions that have undergone the surface treatment to bind to single-stranded nucleoside molecules and to prevent the surface of regions other than the plurality of separated positions from binding to single-stranded nucleoside molecules, thereby limiting the region and density of the distribution of the single-stranded nucleic acid amplification clusters. The form and the result of the surface treatment are not limited as long as the position and density of the single-stranded nucleic acid amplification clusters on the surface of the solid substrate can be defined. Illustratively, reactive groups capable of binding to the single-stranded nucleoside molecules may be formed at a part of the separated positions, or probes that are complementary to a partial sequence of the single-stranded nucleoside molecules may be linked to a part of the separated positions. In some examples, the density of the single-stranded nucleic acid amplification clusters arranged on the surface of the solid substrate is 5 to 800/mm². Illustratively, the density of the single-stranded nucleic acid amplification clusters arranged on the surface of the solid substrate may be 5 to 500/mm², 50 to 500/mm², 100 to 500/mm², 100 to 600/mm², 100 to 700/mm², 100 to 800/mm², 200 to 500/mm², 200 to 600/mm², 200 to 700/mm², 200 to 800/mm², 300 to 500/mm², 300 to 600/mm², 300 to 700/mm², 300 to 800/mm², 400 to 500/mm², 400 to 600/mm², 400 to 700/mm², 400 to 800/mm², 500 to 600/mm², 500 to 700/mm², 500 to 800/mm², 600 to 700/mm², 600 to 800/mm², 700 to 800/mm², etc.

In some certain embodiments, the copy number of the single-stranded nucleic acid molecules of the single-stranded nucleic acid amplification cluster in the surface of the solid substrate may be 10 to 10⁵ polynucleotides. Illustratively, the copy number of the single-stranded nucleic acid molecules of the single-stranded nucleic acid amplification cluster in the surface of the solid substrate may be 10, 20, 30, 40, 50, 60, 70, 80, 90, 100, 500, 800, 1000, 2000, 5000, 8000, 10000, 20000, 50000, 80000, 100000, etc. As such, the density of the single-stranded nucleic acid amplification clusters of the surface of the solid substrate may be up to 10 nmol/L or greater after release.

In the examples of the present application, nucleotide sequences of different single-stranded nucleic acid amplification clusters are different. At least, the single-stranded nucleic acid molecule comprises at least a barcode sequence with a known sequence. That is, the barcode sequences of different single-stranded nucleic acid amplification clusters are unique sequences, such that the single-stranded nucleic acid amplification cluster has a known physical coordinate set relative to the spatial chip 11. The positions of the single-stranded nucleic acid amplification clusters in the surface of the solid substrate are in one-to-one correspondence with the barcode sequences of the single-stranded nucleic acid molecules. As such, after the signal acquired by the detection reaction is acquired, the barcode sequence of the single-stranded nucleic acid amplification cluster can be acquired on the basis of the physical coordinate set of the single-stranded nucleic acid amplification cluster, and other sequence regions of the polynucleotide linked the barcode sequence can be further acquired. On this basis, the analysis and result determination of the detection reaction are achieved.

S40, sequencing the single-stranded nucleic acid amplification cluster to acquire the barcode sequence of each of the single-stranded nucleic acid amplification clusters and a physical coordinate set of each of the single-stranded nucleic acid amplification clusters on a surface of the chip substrate, so as to give the spatial chip. In the examples of the present application, the sequencing of the single-stranded nucleic acid amplification cluster is not strictly limited, and the sequencing may be conducted by, for example, an SBS method to acquire the barcode sequence of each of the single-stranded nucleic acid amplification clusters and a physical coordinate set of each of the single-stranded nucleic acid amplification clusters on a surface of the chip substrate, and determining information that the barcode sequences of the first single-stranded nucleotide library are in one-to-one correspondence with the physical coordinate sets, so as to give the spatial chip.

In some embodiments, the surface of the solid substrate is derived from a chip mother substrate having an area 2 or more times a surface area of the spatial chip, and the method further comprises, before the spatial chip is given:
S50, cutting a surface of the chip mother substrate into a chip substrate with a preset size.

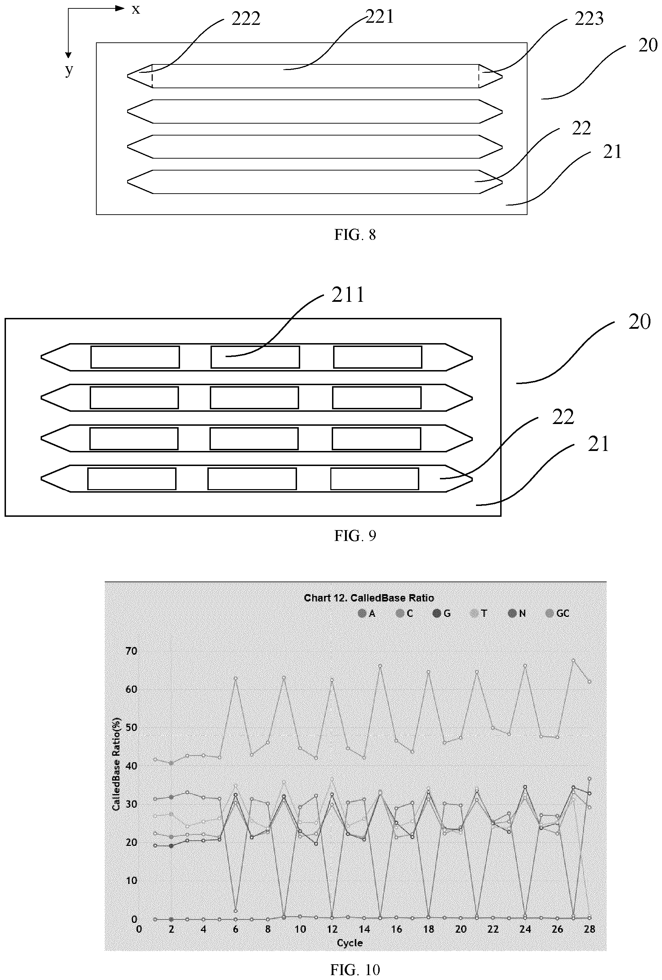

In one embodiment, referring to FIG. 8, the chip mother substrate 20 comprises a second substrate 21, and the second substrate 21 is used for carrying a sample of interest such as a nucleic acid molecule of interest, providing a reaction site for a reaction on the surface, and providing a flow channel for reagents participating in the reaction.

The overall shape of the second substrate 21 may be configured into a shape matching the chip mother substrate 20. Illustratively, the overall shape of the second substrate 21 may be a rectangle, a square, a diamond, another polygon, a circle, an ellipse, or an irregular shape with an arc-shaped circumference. In some examples, the overall shape of the second substrate 21 is configured into a rectangle to fit the structures for receiving and fixing the chip mother substrate 20 in most sequencing platform apparatuses.

The material of the second substrate 21 includes a glass material, such that the second substrate 21 satisfies the requirement for surface flatness. Certainly, the material of the second substrate 21 may also include at least one of silicon dioxide, crystal and quartz glass, or plastic, ceramic, polyethylene terephthalate (PET), poly(methyl methacrylate) (PMMA) or any other suitable material.

In one embodiment, the second substrate 21 is made of a material selected from a transparent material, such that an optical system can acquire the optical signals generated on the surface of the chip mother substrate 20. As such, the type of reactions on the surface of the chip mother substrate 20, in particular, the second substrate 21, can thus be broadened to optical applications. Illustratively, when a substrate having a fluorophore is used to react with a nucleic acid molecule immobilized on the surface of the second substrate 21, the reaction site and the type of the substrate can be determined by the optical signal generated via the fluorophore on the surface of the second substrate 21 acquired by the optical system. Correspondingly, the surface of the second substrate 21 corresponding to the optical system is made of a transparent material including, but not limited to, glass or crystal.

The second substrate 21 is provided with at least one fluid channel 22. The fluid channel 22 is a channel in the chip mother substrate 20 allowing a reagent solution to flow through and also defining a reaction region such as a sequencing region in the chip mother substrate 20. In the examples of the present application, a sample of interest is immobilized on at least a part of the surface of the fluid channel 22. For example, a polynucleotide of interest is immobilized in the fluid channel 22, and a reagent solution reacts with the polynucleotide of interest when flowing through the fluid channel 22. Therefore, the fluid channel 22 also provides a reaction site for the surface reaction of the chip mother substrate 20.

In the examples of the present application, the fluid channel 22 may have an irregular shape or a regular shape. In one embodiment, the dimension of the fluid channel 22 in a first direction is greater than the dimension thereof in a second direction, where the first direction is perpendicular to the second direction, and both the first direction and the second direction are perpendicular to a thickness direction of the second substrate 21. As such, the general shape of the fluid channels 22 formed in the second substrate 21 is defined, which facilitates the control of the fluid in the fluid channels 22, and the positioning and imaging of such region of the chip mother substrate 20. Referring to FIG. 8, the first direction may be direction x, and the second direction may be direction y.

Specifically, for example, when the length of the second substrate 21 is greater than the width, the first direction x may be the length direction of the second substrate 21, and the second direction y may be the width direction of the second substrate 21, such that the dimension of the fluid channel 22 in the first direction x is greater than the dimension thereof in the second direction y, where the first direction is perpendicular to the second direction, and both the first direction and the second direction are further perpendicular to a thickness direction of the second substrate 21.

Similarly, when the length of the second substrate 21 is less than the width, the first direction x may be the length direction of the second substrate 21, and the second direction y may be the width direction of the second substrate 21, such that the dimension of the fluid channel 22 in the first direction x is less than the dimension thereof in the second direction y, where the first direction is still perpendicular to the second direction, and both the first direction and the second direction are still perpendicular to a thickness direction of the second substrate 21.

In some certain embodiments, a plurality of fluid channels 22 are arranged in the chip mother substrate 20, and the fluid channels 22 extend in the first direction; and/or the fluid channels 22 are arrayed in the second direction. As such, the plurality of fluid channels 22 can make the sequencing process more efficient, which facilitates the control of the fluid in the fluid channels 22, and the positioning and imaging of such region of the chip mother substrate 20.

Specifically, the arrangement is that the fluid channels 22 extend in the first direction x, i.e., the length direction of the second substrate 21, and are arrayed in the second direction y, i.e., the width direction of the second substrate 21. Specifically, arranging the plurality of fluid channels 22 can make the sequencing process more efficient. Also, the array arrangement makes the fluid channels 22 uniformly spaced apart, such that the fluid such as the reaction reagent pumped into the fluid channels 22 is uniform.

In some certain embodiments, the fluid channel 22 comprises an intermediate section 221, a first end 222, and a second end 223. The first end 222 and the second end 223 are located at the two ends of the intermediate section 221, respectively, and the dimension of the first end 222 in the second direction and/or the dimension of the second end 223 in the second direction are smaller than the dimension of the intermediate section 221 in the second direction.

In particular, the shape of the fluid channel 22 may be irregular. For example, the fluid channel 22 may comprise the intermediate section 221, the first end 222, and the second end 223. The first end 222 and the second end 223 are symmetrically arranged at the two ends of the fluid channel 22 and are both in a triangular shape. The intermediate section 221 is in the shape of an elongated rectangle, which means that the dimensions of the first end 222 and the second end 223 in the second direction y are smaller than that of the intermediate section 221 in the second direction y.

Certainly, the first end 222 and the second end 223 may also have different shapes, as long as the first end 222 and the second end 223 are located at the two ends of the intermediate section 221, respectively, and the dimension of one of the two in the second direction y is smaller than the dimension of the intermediate section 221 in the second direction y.

In the chip mother substrate 20 provided by the examples of the present application, the surface of the fluid channel 22 is provided with sites. The sites can be interpreted as several separated positions on the second substrate 21, and generally, a sample under detection or a sample of interest (such as polynucleotides, e.g., a single-stranded nucleic acid molecule cluster) may be bonded to the position(s), and a reaction based on the sample can be conducted at the position(s).

It will be appreciated that, in the spatial chip provided above, the micro/nano structures (e.g., 11-3 in FIG. 1) in the chip substrate (which may be interpreted as the first substrate) correspond to the sites in the chip mother substrate 20, and each of the micro/nano structures in the chip substrate may denote one site. In this example, the arrangement and density of the micro/nano structures in the chip substrate are consistent with the arrangement and density of the sites on the chip mother substrate 20.

In the examples of the present application, the distribution of sites in the fluid channel 22 may be random. As one possible implementation, the sites are arrayed in the fluid channel 22. As such, when the detection of the reaction at the regularly arranged sites may provide results with better recognizability and favorably improved sequencing throughput.

Since the chip substrate in the spatial chip is derived from the second substrate 21, for the shape and arrangement of the sites on the second substrate 21, reference can be made to the chip substrate 11 about the micro/nano structures portion of and the arrangement of the single-stranded nucleic acid amplification clusters on the chip substrate, which will not be recited here for brevity.

In the examples of the present application, the chip mother substrate 20 is a mother substrate of the chip substrate, and more specifically, the chip substrate is derived from a region corresponding to the fluid channel 22 in the chip mother substrate. Therefore, in the examples of the present application, the fluid channel 22 has been subjected to the above-mentioned steps S10, S20, S30, and "sequencing the single-stranded nucleic acid amplification cluster to acquire the barcode sequence of each of the single-stranded nucleic acid amplification clusters and a physical coordinate set of each of the single-stranded nucleic acid amplification clusters, and determining information that the barcode sequences of the first single-stranded nucleotide library are in one-to-one correspondence with the physical coordinate sets" in S40, and possesses the features of the chip substrate described above.

In some examples, the step of cutting a surface of the chip mother substrate into a chip substrate with a preset size comprises:
determining one or more preset chip regions on the surface of the chip mother substrate according to a preset shape and the preset size, and marking the preset chip region; and
separating the preset chip region from the chip mother substrate to give the spatial chip.

In this step, referring to FIG. 9, one or more preset chip regions 211 are formed on the surface of the chip mother substrate 20 according to the shape and size of the required chip substrate. It will be appreciated that, in the examples of the present application, the part acquired from the preset chip region 211 corresponds to the chip substrate that constitutes the spatial chip. That is, it will be appreciated that the preset chip region 211 corresponds to a region on the chip mother substrate when the cut of the chip substrate in the spatial chip is not completed yet. Therefore, a preset chip defined by the preset chip region 211 also comprises the first surface and the second surface, and the surface comprising the sites (i.e., having the nucleic acid amplification clusters) is used as the first surface.

In some certain embodiments, when one or more preset chip regions 211 on the surface of the chip mother substrate are determined according to a preset shape and a preset size, the distance between adjacent preset chip regions 211 is greater than or equal to 1 mm. Illustratively, the distance between the preset chip regions 211 is greater than or equal to 2 mm, 3 mm, 4 mm, 5 mm, 6 mm, 7 mm, 8 mm, 9 mm, etc. At this point, when the preset chip region 211 is separated from the chip mother substrate 20, the interference with the adjacent preset chip regions 211 can be reduced, and the influence on the integrity and functionality of the preset chip regions 211 can be reduced. It will be appreciated that a greater distance between adjacent preset chip regions 211 leads to a smaller influence on each other, but a correspondingly lower utilization rate in preparing the chip substrate using the chip mother substrate 20. In some examples, the distance between adjacent preset chip regions 211 is greater than or equal to 9 mm. When the distance between adjacent preset chip regions 211 meets the requirement, simultaneous separation of multiple adjacent preset chip regions 211 can be achieved without interference with each other.

In some examples, separating the preset chip region from the chip mother substrate to give the spatial chip comprises: cutting and separating the preset chip region from the chip mother substrate by laser according to a preset cutting line. In some examples, the distance between adjacent preset cutting lines is greater than or equal to 2 mm, that is, the distance between adjacent preset chip regions 211 is greater than or equal to 2 mm. The method provided by the examples of the present application comprises the step of marking the determined preset chip region 211. By setting the marker on the surface where the polynucleotide is located, the arrangement direction of the chip substrate defined by the preset chip region 211 and the source of the chip substrate can be acquired, and furthermore, the site information in the chip substrate, including first positional information of the sites and the barcode sequence corresponding to each positional information, is acquired. Therefore, when the acquired chip substrate is used in spatial omics, when the spatial chip is used in a biochemical assay, the site information in the chip substrate can be quickly acquired through the markers, thus achieving spatial omics detection.

In some certain embodiments, for the markers formed by the marking process, reference can be made to the above description about the type and manner of the markers on the surface of the chip substrate.

In some examples, the marking process comprises forming one or more markers in the preset chip region 211. In one embodiment, the surface of the preset chip region 211 to which the polynucleotides are bonded (corresponding to the first surface of the chip substrate) is marked to give one or more markers. In one embodiment, the second substrate 21 is provided with one or more markers for marking the orientation of the chip substrate (in particular, when the chip substrate is arranged on the surface of the solid carrier, the orientation of the chip substrate relative to the solid carrier can be determined by the second substrate 21) and the location of the region in the chip substrate.

In the examples of the present application, the marking mode of the marker is not limited strictly, and in some certain embodiments, one of a letter, a number and a graphic, or a combination thereof may be used. Illustratively, a marker of a letter alone, a marker of a number alone, a marker of a graphic alone, a marker comprising a letter and a number, a marker comprising a letter and a graphic, or a marker comprising a number and a graphic, or a marker comprising a letter, a number and a graphic may be used.

In some examples, the marker includes a directional marker and a regional marker. The directional marker is configured for indicating the orientation of the chip substrate bonded to the surface of the solid carrier, such that the site information on the chip substrate can be accurately positioned in the subsequent detection. In one example, the directional marker may be an arrow marker to indicate the direction of the chip substrate.

In the examples of the present application, one chip substrate may be provided with one or more directional markers. The positions for arranging the directional markers on the second substrate 21 may be outside the region where the sites are arranged on the second substrate 21. In some examples, when the chip substrate is a rectangular chip, the directional marker may be arranged in regions where one or more right angles of the second substrate 21 are located, so as to quickly distinguish the direction of the chip substrate.

In some examples, each of the chip substrates may comprise one or more regional markers. When one regional marker is arranged on the second substrate 21 of one chip substrate, for the detection reaction performed in each reaction region, the regional marker is used as a reference for position calibration to determine the site information (including first positional information of the sites and the barcode sequence corresponding to each positional information) of the reaction region, and then the signal or result of the corresponding detection reaction is acquired and analyzed.

Thus, in some examples, the surface of the preset chip region 211 to which the polynucleotide is bonded is provided with a plurality of regional markers. By arranging the plurality of regional markers, regions on the surface of the chip substrate can be separately marked, such that when the surface of the chip substrate is subjected to a detection reaction, the relative or absolute position of the reaction region in the chip substrate can be quickly learned via the regional markers, thus acquiring the corresponding site information. When a detection reaction is performed using the chip substrate, this method, on the basis of the current regional marker, can quickly achieve region calibration, quickly determine the site information (including first positional information of the sites and the barcode sequence corresponding to each positional information) of the region, and acquire a signal or a result of the corresponding detection reaction, thereby avoiding repeated calibration when using one mark as a reference for each reaction.

In the examples of the present application, when the surface of the preset chip region 211 to which the polynucleotide is bonded is provided with the plurality of regional markers, the plurality of regional markers may be achieved by using different marker patterns, such as: different letters/numbers (including different letters and/or cases, such as A, B, C, D, a, b, c, d, etc.), different patterns, such as circles, triangles, rectangles, squares, ellipses, other polygons, etc., or different letters and patterns, etc. Certainly, the marker patterns may be distinguished by the arrangement at different arrangement angles relative to one direction of the first surface 1211 (such as the orientation of the vertex angle of a non-equilateral triangle, etc.), or may be distinguished by using different numbers of marker patterns (such as a square or a triangle or a circle, two squares or triangles or circles regularly arranged in a certain arrangement rule, etc.).

In some examples, the plurality of regional markers are arranged close to the circumference of the first substrate 21. In some examples, the first substrate 21 has a polygonal shape, and the regional markers are uniformly distributed in a direction parallel to each side of the polygon. In one example, for the convenience of quick recognition, the types of the regional markers are different for markers arranged on different sides. For example, the four sides of a rectangle are respectively marked with circles, triangles, squares, and rectangles. In another example, the first substrate 21 is circular or elliptical, and the regional markers are uniformly distributed along a track close to the circumference. By uniformly dispersing the plurality of regional markers, the speed of positioning the detection region is increased, thereby improving the efficiency of the detection reaction and analysis of the spatial chip.

In one embodiment of the present application, the marker further includes a chip serial number marker for distinguishing different chip substrates and making the arrangement order of the chip substrates on the solid carrier traceable. The marker also includes a chip source marker. By source marker on the preset chip region 211, i.e., recording the positions and regions of the sites 1213 contained in the preset chip region 211 in the chip mother substrate 20, a unique barcode sequence corresponding to each of the sites contained in the preset chip region 211 is determined. In some examples of the present application, the chip source marker is a combination of a number and a letter. For example, in 3B, "3" denotes a first fluid channel derived from the chip mother substrate 20, and "B" denotes a second division in the first fluid channel. Illustratively, when the mother chip is provided with four sequencing regions such as four sequencing channels, and three chip substrates 12 are sequentially separated in a certain direction in each sequencing region, 3B may denote the second chip substrate 12 separated in a preset direction from the third fluid channel of the large source chip. In some examples, marking the preset chip region may be conducted by laser marking or by printing using a label printer.

In some examples, when the spatial chip comprises a plurality of chip substrates, the method further comprises: S60, fixing one or more chip substrates on a surface of a solid carrier in a preset arrangement.

In this step, the selection of the solid carrier is described above and will not be recited here for brevity.

In some certain embodiments, when the chip substrate is fixed on the surface of the solid carrier, a certain space is arranged between the edge of the chip substrate and the solid-phase carrier, so as to avoid abrasion of or damage to the edge of the chip substrate. In some examples, a certain space is arranged between the edge of the chip substrate and the solid carrier, so as to avoid abrasion of or damage to the edge of the chip substrate during the processes of preparation, storage and transportation and thus the loss of site data. In some certain embodiments, the distance between the edge of the chip substrate and the edge of the solid carrier is greater than or equal to 1 mm. Illustratively, the distance between the edge of the chip substrate and the edge of the solid carrier is greater than or equal to 1 mm, 2 mm, 3 mm, 4 mm, 5 mm, 6 mm, etc., without limitation. In some examples, the distance between the edges of adjacent chip substrate and solid carrier is one quarter or more of the length of the chip substrate in the direction of the distance.

In the examples of the present application, one chip substrate may be arranged on one solid carrier, or two or more chip substrates may be arranged simultaneously. The number and density of the chip substrates on the solid carrier can be determined in accordance with actual requirements. In some examples, the distance between adjacent chip substrates is greater than or equal to 1 mm. Setting an appropriate distance between the adjacent chip substrates can avoid cross-interference when conducting the detection reactions and thus detection crosstalk when performing signal acquisition or detection on the chip substrates after the reaction. It will be appreciated that when two or more chip substrates are simultaneously arranged on one solid carrier, a plurality of chip substrates may be arrayed on the surface of the solid carrier. In some examples, each of the chip substrates has known second positional information relative to the solid carrier. The second positional information is acquired from the chip serial number marker of the second substrate 21.

In the examples of the present application, the chip substrate is fixed on the solid carrier via the second surface. The bonding mode of the second surface to the solid carrier is not strictly limited. For example: the second surface and the solid carrier can be bonded by electrostatic adsorption; the second surface and the surface of the solid carrier are subjected to surface chemical modification, and the two are bonding chemically; or the chip substrate may also be fixed to the surface of the solid carrier by providing a glue layer between the second surface and the solid carrier. Certainly, the bonding mode of the second surface to the solid carrier is not limited to those above, and theoretically, any bonding mode capable of fixing the chip substrate to the solid carrier should construed within the scope of the present application.

In some embodiments, a glue layer is arranged between the second surface and the solid carrier, and the chip substrate is fixed by the adhesive action of the glue layer. The glue layer may cover the entire second surface to completely bond the second surface to the solid carrier surface, or the glue layer may be arranged on a part of the area of the second surface to partially adhere the second surface to the surface of the solid carrier. It will be appreciated that when using the same adhesive material and adhesion mode, a larger adhered area may result in a firmer bonding between the second surface and the solid carrier. When the glue layer is arranged on a part of the area of the second surface, the area where the glue layer is arranged may be an edge area, or may be several bonding regions uniformly or non-uniformly arranged on the glue layer.

In some examples, the glue layer is made of a material having a light transmittance of greater than or equal to 90%, such as an optical adhesive, etc. As such, the obtained spatial chip can be used for optical detection without being affected by the light transmittance of the glue layer. Illustratively, the glue layer is at least one selected from a transparent double-sided adhesive, an epoxy adhesive such as an epoxy resin, and an acrylate adhesive such as an acrylic resin.

In some certain embodiments, a double-sided tape is applied to a preset region on the surface of the solid carrier, and the chip substrate is attached to the surface of the solid carrier to form the spatial chip.

In some certain embodiments, a chip label is formed on the surface of the solid carrier; the chip label is a computer-storable information label, and the chip label is a QR code or a barcode or other labels or instructions that can be associated with computer-storable information. By scanning the QR code or the barcode, the positions of the sites included in the chip substrate and the unique barcode sequence corresponding to each position can be quickly acquired.

After that, the obtained spatial chip may be packaged and preserved. The examples of the present application further provide use of the spatial chipdescribed above or the spatial chip provided by the method described above in the field of spatial omics.

In some embodiments, a composition is provided, comprising a spatial chip comprising one or more chip substrates having at least one surface provided with a plurality of isolated single-stranded nucleic acid amplification clusters, where single-stranded nucleic acid molecules in the single-stranded nucleic acid amplification cluster have identical nucleotide sequences, and nucleotide sequences of different single-stranded nucleic acid amplification clusters are different;

The single-stranded nucleic acid amplification cluster has a known physical coordinate set relative to the spatial chip. As shown in FIG. 7, the single-stranded nucleic acid molecule 111-1 comprises a barcode sequence B with a known sequence and a first capture sequence A and a second capture sequence C1 respectively linked to the two ends of the barcode sequence, where the first capture sequence A is linked to a surface of the chip substrate 11, and the barcode sequences B of the first single-stranded nucleotide molecules 111-1 are in one-to-one correspondence with the physical coordinate sets.

In some examples, the spatial chip is the spatial chip described above. In some examples, referring to FIG. 7, the composition further comprises a second single-stranded nucleotide library 2. The second single-stranded nucleotide library 2 comprises a first base sequence C2, a second base sequence D, and a capture part E; the first base sequence C2 is a known sequence, the second base sequence D is a label sequence, and the first base sequences C2 of the nucleotides in the second single-stranded nucleotide library 2 are identical, but the label sequences are different.

In some embodiments, the first base sequence C2, the second base sequence D, and the capture part E are linked in sequence, where the first base sequence C2 is complementary to the second capture sequence C1, and the capture part E is a primer sequence. At this point, the composition can be used for spatial reverse transcription analysis. Specifically, the second single-stranded nucleotide library 2 can bind to the first single-stranded nucleic acid molecule 111-1 via the first base sequence C2 and the second capture sequence C1, and RNA in a sample of interest, such as a cell sample or a tissue sample, can be captured by the primer sequence provided by the capture part E. After the characteristic detection of the captured sample by characteristic analysis such as fluorescence analysis, the reverse transcription information of the detected characteristic in the sample is restored from the sequencing information of barcode B in the first single-stranded nucleic acid molecule 111-1.

In some examples, the primer sequence is poly-dT, poly-dA, a random primer, or a target molecule primer, which can be configured according to the spatial chip application scene.

In some embodiments, the capture part E is a transposase, and the first base sequence C2 and the second base sequence D are located in the transposase. At this point, the spatial chip can be used for ATAC sequencing analysis.

In some embodiments, the first base sequence C2, the second base sequence D, and the capture part E are linked in sequence, where the first base sequence C2 is complementary to the second capture sequence C1, and the capture part E is a nucleic acid aptamer, a protein, an enzyme, or an antibody. At this point, the spatial chip can be used for spatial protein analysis. Specifically, the second single-stranded nucleotide library 2 can bind to the first single-stranded nucleic acid molecule 111-1 via the first base sequence C2 and the second capture sequence C1, and a sample of interest, such as a cell sample or a tissue sample, can be captured by the primer sequence provided by the capture part E. After the characteristic detection of the captured sample by characteristic analysis such as fluorescence analysis, the spatial information of the detected characteristic in the sample is restored from the sequencing information of the barcode in the first single-stranded nucleic acid molecule.

In one embodiment, a method for spatial reverse transcription is provided, comprising:
(51) Providing a spatial chip and a second single-stranded nucleotide library.

Referring to the description about the spatial chip, the spatial chip comprises a chip substrate; at least one surface of the chip substrate is provided with a plurality of isolated single-stranded nucleic acid amplification clusters; single-stranded nucleic acid molecules in the single-stranded nucleic acid amplification cluster have identical nucleotide sequences, and nucleotide sequences of different single-stranded nucleic acid amplification clusters are different; the single-stranded nucleic acid amplification cluster has a known physical coordinate set relative to the spatial chip, and the single-stranded nucleic acid molecule comprises a barcode sequence with a known sequence and a first capture sequence and a second capture sequence respectively linked to the two ends of the barcode sequence; the first capture sequence is linked to a surface of the chip substrate, and the barcode sequences of each single-stranded nucleic acid amplification clusters are in one-to-one correspondence with the physical coordinate sets. Other aspects of the spatial chip are described above and will not be recited here for brevity.

The second single-stranded nucleotide library comprises a first base sequence, a second base sequence and a capture part that are linked in sequence; the first base sequence is a known sequence complementary with the second capture sequence, the second base sequence is a label sequence, the capture part is a primer sequence, and the first base sequences of the nucleotides in the second single-stranded nucleotide library are identical, but the label sequences are different.

(52) Contacting a cell or tissue sample of interest with the spatial chip and the second single-stranded nucleotide library to bond RNA in the cell or tissue sample of interest on the spatial chip via the second single-stranded nucleotide library.

In this step, contacting the cell or tissue sample of interest with the spatial chip and the second single-stranded nucleotide library can be achieved by adding the cell or tissue sample of interest and the second single-stranded nucleotide library to the spatial chip, and the addition mode includes introduction and dropwise addition. In one embodiment, the step of contacting the cell or tissue sample of interest with the spatial chip and the second single-stranded nucleotide library comprises: contacting the cell or tissue sample of interest with the spatial chip, and then adding the second single-stranded nucleotide library. The two are linked by the two ends of the second single-stranded nucleotide library. In another embodiment, the second single-stranded nucleotide library is added to the spatial chip to bind the second single-stranded nucleotide library to the first single-stranded nucleic acid molecule on the surface of the spatial chip, and then the cell or tissue sample of interest is added.

In some examples, when the cell or tissue sample of interest is a tissue sample, the tissue sample is a tissue section. In some examples, the tissue section has a thickness of less than or equal to 50 µm.

The second single-stranded nucleotide library can bind to the first single-stranded nucleic acid molecule via the first base sequence and the second capture sequence, and by capturing RNA in the cell or tissue sample of interest with the primer sequence provided by the capture part, the RNA in the cell or tissue sample of interest binds to the spatial chip via the second single-stranded nucleotide library.

In this step, the captured RNA may be subjected to reverse transcription to give cDNA. In some examples, after the characteristic detection of the reverse transcription product by characteristic analysis such as fluorescence analysis, corresponding detection characteristics such as fluorescence signals are acquired. (53) Cleaving the spatial chip, and collecting a cleavage product.

In this step, the first capture sequence of the spatial chip is cleaved to give the cleavage product. In one embodiment, if the captured RNA is subjected to reverse transcription in step (52), the cleavage product comprises the double-stranded molecule formed by the single-stranded nucleic acid molecule-second nucleic acid molecule-RNA and cDNA after the removal of the first capture sequence.

In another embodiment, if the captured RNA is not subjected to reverse transcription in step (52), the cleavage product comprises the single-stranded nucleic acid molecule-second nucleic acid molecule-RNA after the removal of the first capture sequence. Furthermore, the RNA in the cleavage product may be subjected to reverse transcription to give cDNA. In some examples, after the characteristic detection of the reverse transcription product by characteristic analysis such as fluorescence analysis, corresponding detection characteristics such as fluorescence signals are acquired.

(54) Sequencing the cleavage product, and analyzing obtained sequencing data.

In this step, the cleavage product is sequenced to give the sequence information of the barcode, and the spatial information of the detected characteristic in the sample can be restored for the positional information in the spatial chip corresponding to the barcode in the first single-stranded nucleic acid molecule.

In some examples, analyzing the obtained sequencing data comprises:
on the basis of the sequencing data, acquiring positional information in the spatial chip of the barcode sequence in the cleavage product; and
on the basis of the positional information, performing spatial restoration on the cleavage product to achieve spatial reverse transcription analysis.

In one embodiment, a method for ATAC sequencing is provided, comprising:
(61) Providing a spatial chip and a second single-stranded nucleotide library.

Referring to the description about the spatial chip, the spatial chip comprises a chip substrate; at least one surface of the chip substrate is provided with a plurality of isolated single-stranded nucleic acid amplification clusters; single-stranded nucleic acid molecules in the single-stranded nucleic acid amplification cluster have identical nucleotide sequences, and nucleotide sequences of different single-stranded nucleic acid amplification clusters are different; the single-stranded nucleic acid amplification cluster has a known physical coordinate set relative to the spatial chip, and the single-stranded nucleic acid molecule comprises a barcode sequence with a known sequence and a first capture sequence and a second capture sequence respectively linked to the two ends of the barcode sequence; the first capture sequence is linked to a surface of the chip substrate, and the barcode sequences of each single-stranded nucleic acid amplification clusters are in one-to-one correspondence with the physical coordinate sets. Other aspects of the spatial chip are described above and will not be recited here for brevity.

The second single-stranded nucleotide library comprises a first base sequence, a second base sequence, and a capture part; the first base sequence is a known sequence, the second base sequence is a label sequence, and the first base sequences of the nucleotides in the second single-stranded nucleotide library are identical, but the label sequences are different; the capture part is a transposase, and the first base sequence and the second base sequence are located in the transposase.

(62) Contacting a cell or tissue sample of interest with the spatial chip and the second single-stranded nucleotide library to bond ATAC in the cell or tissue sample of interest on the spatial chip via the second single-stranded nucleotide library.

In this step, contacting the cell or tissue sample of interest with the spatial chip and the second single-stranded nucleotide library can be achieved by adding the cell or tissue sample of interest and the second single-stranded nucleotide library to the spatial chip, and the addition mode includes introduction and dropwise addition. In one embodiment, the step of contacting the cell or tissue sample of interest with the spatial chip and the second single-stranded nucleotide library comprises: contacting the cell or tissue sample of interest with the spatial chip, and then adding the second single-stranded nucleotide library. The two are linked by the two ends of the second single-stranded nucleotide library. In another embodiment, the second single-stranded nucleotide library is added to the spatial chip to bind the second single-stranded nucleotide library to the first single-stranded nucleic acid molecule on the surface of the spatial chip, and then the cell or tissue sample of interest is added.

In some examples, when the cell or tissue sample of interest is a tissue sample, the tissue sample is a tissue section. In some examples, the tissue section has a thickness of less than or equal to 50 µm.

(63) Cleaving the spatial chip, and collecting a cleavage product.

In this step, the first capture sequence of the spatial chip is cleaved to give the cleavage product.
(64) Sequencing the cleavage product, and analyzing obtained sequencing data.

In this step, the cleavage product is sequenced to give the sequence information of the barcode, and the spatial information of the detected characteristic in the sample can be restored for the positional information in the spatial chip corresponding to the barcode in the first single-stranded nucleic acid molecule.

In some examples, analyzing the obtained sequencing data comprises:
on the basis of the sequencing data, acquiring positional information in the spatial chip of the barcode sequence in the cleavage product; and
on the basis of the positional information, performing spatial restoration on the cleavage product to achieve ATAC sequencing analysis.

In one embodiment, a method for spatial protein analysis is provided, comprising:
(71) Providing a spatial chip and a second single-stranded nucleotide library.

Referring to the description about the spatial chip, the spatial chip comprises a chip substrate; at least one surface of the chip substrate is provided with a plurality of isolated single-stranded nucleic acid amplification clusters; single-stranded nucleic acid molecules in the single-stranded nucleic acid amplification cluster have identical nucleotide sequences, and nucleotide sequences of different single-stranded nucleic acid amplification clusters are different; the single-stranded nucleic acid amplification cluster has a known physical coordinate set relative to the spatial chip, and the single-stranded nucleic acid molecule comprises a barcode sequence with a known sequence and a first capture sequence and a second capture sequence respectively linked to the two ends of the barcode sequence; the first capture sequence is linked to a surface of the chip substrate, and the barcode sequences of each single-stranded nucleic acid amplification clusters are in one-to-one correspondence with the physical coordinate sets. Other aspects of the spatial chip are described above and will not be recited here for brevity.

The second single-stranded nucleotide library comprises a first base sequence, a second base sequence and a capture part that are linked in sequence; the first base sequence is a known sequence complementary with the second capture sequence, the second base sequence is a label sequence, and the first base sequences of the nucleotides in the second single-stranded nucleotide library are identical, but the label sequences are different. In some examples, the capture part is a nucleic acid aptamer, a protein, an enzyme, or an antibody.
(72) Contacting a cell or tissue sample of interest with the spatial chip and the second single-stranded nucleotide library to bond a target having a binding capacity with the capture part in the sample of interest on the spatial chip via the second single-stranded nucleotide library.

In this step, contacting the cell or tissue sample of interest with the spatial chip and the second single-stranded nucleotide library can be achieved by adding the cell or tissue sample of interest and the second single-stranded nucleotide library to the spatial chip, and the addition mode includes introduction and dropwise addition. In one embodiment, the step of contacting the cell or tissue sample of interest with the spatial chip and the second single-stranded nucleotide library comprises: contacting the cell or tissue sample of interest with the spatial chip, and then adding the second single-stranded nucleotide library. The two are linked by the two ends of the second single-stranded nucleotide library. In another embodiment, the second single-stranded nucleotide library is added to the spatial chip to bind the second single-stranded nucleotide library to the first single-stranded nucleic acid molecule on the surface of the spatial chip, and then the cell or tissue sample of interest is added.

In some examples, when the cell or tissue sample of interest is a tissue sample, the tissue sample is a tissue section. In some examples, the tissue section has a thickness of less than or equal to 50 µm.

(73) Cleaving the spatial chip, and collecting a cleavage product.

In this step, the first capture sequence of the spatial chip is cleaved to give the cleavage product.

(74) Sequencing the cleavage product, and analyzing obtained sequencing data.

In this step, the cleavage product is sequenced to give the sequence information of the barcode, and the spatial information of the detected characteristic in the sample can be restored for the positional information in the spatial chip corresponding to the barcode in the first single-stranded nucleic acid molecule.

In some examples, analyzing the obtained sequencing data comprises:
on the basis of the sequencing data, acquiring positional information in the spatial chip of the barcode sequence in the cleavage product; and
on the basis of the positional information, performing spatial restoration on the cleavage product to achieve spatial protein analysis.

The present application will be illustrated with reference to the following specific examples.

### Example 1

(1) Two libraries with the following sequences were designed, where the barcode sequences are underlined, B denotes a base other than A (one selected from G, C, and T), V denotes a base other than T (one selected from G, C, and A), and N denotes a random base of ACGT with the specific type being unknown:
Library 1: 
5’-
AATGATACGGCGACCACCGAGATCTACACCCAAGCTTGGCTGATAAGGTCGCCATGCCTCTCAGT
ACGTCAGCAGNNNNNBNNBNNBNNBNNBNNBNNBVATCTCGTATGCCGTCTTCTGCTTG
Library 2: 
5’-
AATGATACGGCGACCACCGAGATCTACACBVNNVNNVNNVNNVNNVNNVNNNNNCTGCTG
ACGTACTGAGAGGCATGGCGACCTTATCAGCCAAGCTTGGATCTCGTATGCCGTCTTCTGCTTG
(2) The preparation of the library comprises the following procedures: two nucleotide sequences 1-1 and 1-2 constituting library 1, and two nucleotide sequences 2-1 and 2-2 constituting library 2 were provided; 2 RNA splint oligo primer sequences for connection with 1-1 and 1-2, and 2 RNA splint oligo primer sequences for connection with 2-1 and 2-2 were designed;
(3) For example, for the preparation of library 1, a T4 DNA ligation reaction system was prepared according to Table 1 below to ligate the nucleotide sequences 1-1 and 1-2;

**Table 1**

| Component | Volume (µL) |
|---|---|
| 10×T4 DNA Ligase Buffer (NEB M0202) | 2 |
| T4 DNA ligase (NEB M0202) | 2 |
| 1-1 (Sangon, 100 µM) | 1 |
| 1-2 (Sangon, 100 µM) | 1 |
| RNA splint oligo1 (100 µM) | 2 |
| 50% PEG8000 (YEASEN 60300ES60) | 8 |
| NF-H₂O | 4 |
| Total | 20 |

(4) A PCR amplification system was prepared according to Table 2 below, uniformly mixed after the preparation was finished, and instantly preserved on ice for later use;

**Table 2**

| Component | Volume (µL) |
|---|---|
| 2×KAPA HiFi HotStart ReadyMix (Roche KK2602) | 50 |
| lib_F (10 µM, Sangon) | 5 |
| lib_R (10 µM, Sangon) | 5 |
| dNTP Mix (10 mM each Vazyme P031) | 10 |
| Fragmentation product | 30 |
| Total | 100 |

(5) The amplification system in Table 2 above was used for the PCR reaction performed on the ligation product according to the amplification procedures shown in Table 3 below;

**Table 3**

| Temperature | Time | Number of cycles |
|---|---|---|
| 105 °C | Heated lid | 1 |
| 95 °C | 5 min | |
| 98 °C | 20s | 8 |
| 60 °C | 20s | |
| 72 °C | 30s | |
| 72 °C | 3min | 1 |
| 12 °C | Hold | |

(6) The PCR product of the band at the position of 127 b in 2% agarose electrophoresis was recovered.
(7) The recovered product was subjected to high-throughput sequencing. According to the manual instructions of the sequencing platform, the recovered library was diluted to the required concentration and hybridized to the surface of a sequencing chip. After surface solid amplification and linearization, an SBS reaction of 28 bases was performed on the first strand to give unique barcode information. The sequencing results of a part of clusters in the same FOV are shown in FIG. 10, and the sequence and spatial coordinate information of a part of the clusters are shown in Table 4 below;

**Table 4**

| 28N sequence obtained by sequencing | Spatial coordinates (x:y) |
|---|---|
| CTTTCAATAATGTCATGCTGCTCCCGAC | 1143:1268 |
| GGCTGAAGTAGGTGTAGTCGATTGCGGG | 1154:1396 |
| TTACGTCGGTCAAGCGCGTTGAGAGCTA | 2730:1440 |
| CCCAGTACACTGACCCTTTTGCCGTCTT | 2909:0977 |
| CGATGAATACCGATACCATTATGACCCG | 3391:1297 |
| GCACCCAGACCCTCACCCACATCAGTAC | 1738:1472 |
| ATACTTTTCGGTCTCGCGATTCTGTTTT | 3620:0991 |

(8) The chip channels were washed with formamide at 55 °C and then washed with 5× SSC to remove the formamide;
(9) At 45 °C, the surface clustered library was hybridized with 5 µM HandIII Cut primer for 20 min. A HandIII enzyme digestion system shown in Table 5 below was prepared, introduced into the chip channels, and reacted for 3 h at 37 °C;

**Table 5**

| Component | Volume (µL) |
|---|---|
| HandIII (20u/µL YEASEN 15012ES76) | 10 |
| 10×FuniCut^{™} Buffe (YEASEN 15012ES76) | 10 |
| Nuclease-free H₂O (Invitrogen AM9939) | 80 |
| Total | 100 |

(6) The 3' end of the nucleic acid molecule sequence was exposed and washed with formamide to give a single-stranded nucleic acid molecule probe, which is denoted by: chip substrate-A-B-C1, where A was derived from the immobilizing strand of the modified substrate of the sequencing chip, B + C1 were obtained by the surface solid amplification based on A, B was a sequence of 28 random bases, and C1 was a known sequence of 39-43 bases (containing primer-binding sites).

### Example 2

(1) Preparation of polyA probe strand with aminoallyl-dUTP:
The template strand was an RNA sequence: 
5'NBUUUUUUUUUUUUUUUUUUUUUUUUUUUUUUNNNNNNNNNNNNNNNNGAAGAUACCUGC
GACCAUCCAGUUCCACUCA;
The extension primer was a DNA sequence with a 5-terminal phosphate modification:
   5'P-TGAGTGGAACTGGATGGTCGCAGGTATCTTC;
(2) A reaction system was prepared according to Table 6 below, and the reaction was conducted for 15 min at 55 °C and 20 min at 75 °C:

**Table 6**

| Component | Volume (µL) |
|---|---|
| 5× Maxima RT Buffer (Thermo EP0741) | 4 |
| RNase inhibitor (40u/µL Vazyme R301) | 1 |
| Maxima Reverse Transcriptase (200U/µL Thermo EP0741) | 1 |
| dNTP Mix (10 mM each Vazyme P031) | 2 |
| Aminoallyl-dUTP solution (Thermo, 10 mM) | 0.4 |
| 5M Betaine (Sigma B0300) | 4 |
| 50% PEG20000 (YEASEN 60300ES60) | 2 |
| Template strand RNA (Sangon, 100 µM) | 2 |
| Extension primer (Sangon, 100 µM) | 2 |
| Nuclease-free H₂O (Invitrogen AM9939) | 0.6 |
| Total | 20 |

(3) After sequencing and restriction enzyme digestion, the immobilizing strand was ligated to a polyA probe strand containing an aminoallyl-dUTP. The surface ligation reaction system was prepared according to Table 7:

**Table 7**

| Component | Volume (µL) |
|---|---|
| 10×T4 DNA Ligase Buffer (NEB M0202) | 10 |
| T4 DNA ligase (NEB M0202) | 10 |
| Reaction product of previous step | 20 |
| LigaseCAC2 (100 µM) | 10 |
| 50% PEG8000 (YEASEN 60300ES60) | 40 |
| NF-H₂O | 10 |
| Total | 100 |

| | |
|---|---|
| LigaseCAC2 primer sequence: 5'ATCCAGTTCCACTCATGGCTGATAAGGTCGCC. | |

(4) The above ligation system was loaded on a solid reaction carrier and reacted overnight at 25 °C. The chip channels were washed with formamide, where the surface immobilized cluster was in a single-stranded state.
(5) Fresh brain tissues of mice were embedded in OCT, frozen in a freezer at -80 °C, and thawed to -20 °C before use;
(6) The tissue was sliced into sections with a thickness of 10 µm and attached to the prepared nucleic acid chip. 4% paraformaldehyde was dropwise added for fixation at room temperature for 10 min;
(7) The tissue sections were treated with 0.5% NP-40 PBS at room temperature for 5 min before the buffer was removed;
(8) The nucleic acid chip with the tissue was treated for 30 min with BS(PEG)9 (Thermo, 21582), pH 7.0, prepared with PBS, such that the immobilizing strand probe could be crosslinked with the tissue;
(9) The USER enzyme reaction system was prepared according to Table 8 below:

**Table 8**

| Component | Volume (µL) |
|---|---|
| USER (1U/µL NEB M5505L) | 5 |
| 10×rCutsmart (NEB M5505L) | 10 |
| 50% PEG8000 (YEASEN 60300ES60) | 8 |
| Nuclease-free H₂O (Invitrogen AM9939) | 77 |
| total | 100 |

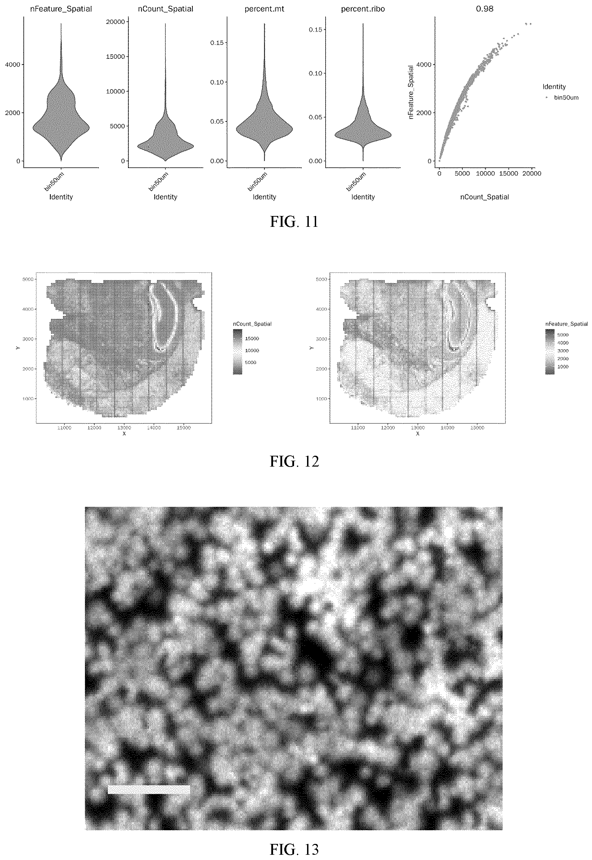

(9) The NP-40 permeabilization system was removed, the USER enzyme digestion system was dropwise added, and the system was reacted for 20 min at 37 °C;
(10) The USER enzyme reaction system was removed, and the product of the previous step was recovered by using KOH;
(11) After a water-in-oil reaction system was established, reverse transcription and cDNA Library construction were conducted by using a Vazyme VAHTS Universal V10 RNA-seq Library Prep Kit (Vazyme, NR606-01);
(12) The product from the previous step was subjected to high-throughput sequencing, and PE100 sequencing was performed on a sequencing platform (PE denotes paired-end sequencing, and 100 denotes that the length of the sequence of interest is 100 bases).
(13) Data comparison and analysis: 28N and 10 UMI information of the first strand in fq obtained by sequencing was extracted, and the 28N data were compared with fq data obtained by the first sequencing in Example 1 to give positional information, where UMI was used for quantifying the gene expression; the cDNA information of the second strand in fq obtained by sequencing was used for gene alignment.
(14) The bin50µm statistics results are shown in FIG. 11. The ordinates of the first two panels denote the number and respectively, the number distribution of bonded genes and UMIs. Panels 3 and 4 show graphs illustrating the log number of the bonded gene types and UMIs, respectively; the abscissa of the last panel denotes the number of bonded UMI and the ordinate denotes the number of bonded gene types. It can be seen that as the number of UMI increased, the identified gene types increased and were eventually saturated.
(15) Taking bin50µm as an example, the sequences of bonded UMIs in the window were detected, and restored to the window according to the positional information corresponding to the sequences, thus giving the left panel of FIG. 12; the Gene types were also restored to the window in accordance with the positional information, thus giving the right panel in FIG. 12. Therefore, the spatial chip provided by the examples of the present application can restore the spatial positional information and achieve tissue spatial transcriptomics analysis.

### Example 3

This example provides a method for preparing a spatial chip having oligodT on the surface thereof, comprising the following procedures:
(1) A library with 28N was designed and synthesized, with the sequence as follows: 
5’-
AATGATACGGCGACCACCGAGATCTACACBVNNVNNVNNVNNVNNVNNVNNVNNNNNCTGCTG
ACGTACTGAGAGGCATGGCGACCTTATCAGCCAAGCTTGGATCTCGTATGCCGTCTTCTGCTTG,
 the barcode sequences are underlined, B denotes a base other than A (GCT), V denotes a base other than T (GCA), and N denotes a random base of ACGT with the specific type being unknown;
(2) According to the manual instructions of the sequencing platform, the library in step (1) was denatured, diluted to 100 pM, transferred to the library wells on the kit, and subjected to SE sequencing (single-end sequencing) to give the barcode sequence;
(3) At 45 °C, the clustered library in step (1) was hybridized with 5 µM HandIII Cut primer for 20 min. A HandIII enzyme digestion system shown in Table 9 below was prepared, uniformly mixed, and preserved on ice for later use;

**Table 9**

| Component | Volume (µL) |
|---|---|
| HandIII (20u/uL YEASEN 15012ES76) | 10 |
| 10×FuniCut^{™} Buffe (YEASEN 15012ES76) | 10 |
| Nuclease-free H₂O (Invitrogen AM9939) | 80 |
| Total | 100 |

(4) The hybridization system was washed with 1× FuniCut^{™} buffer, a HandIII enzyme digestion system was introduced, and the system was reacted for 3 h at 37 °C;
(5) The digested chip was washed with formamide to give a single-stranded nucleic acid molecular probe;
(6) The Klenow extension system shown in Table 10 below was prepared, uniformly mixed, and preserved on ice for later use;

**Table 10**

| Component | Volume (µL) |
|---|---|
| 10 × Blue Buffer (Vazyme N105) | 10 |
| Klenow Fragment exo- (Vazyme N105) | 6 |
| dNTP Mix (10 mM each Vazyme P031) | 14 |
| Extension primer (100 µM) | 10 |
| NF-H₂O | 60 |
| Total | 100 |

The sequence of the extension primer is as follows: 5'NBAAAAAAAAAAAAAAAAAAAAAAAAAAAAAANNNNNNNNNNGGCTGATAAGGTCGCCAT GCCTCTCAGTACGTCAGCAG; (7) The Klenow extension system was introduced into the chip treated in step (5), and extended for 1 h at 37 °C; (8) A 30-A base primer (concentration: 1 µM) with cy3 was hybridized with the oligodT extending from the surface in step (7), and the results of fluorescence detection are shown in FIG. 13. As shown in FIG. 13, the results indicate that the chip demonstrated clustered fluorescence, suggesting a successful surface oligodT extension. The bright bar is a scale of 10 µm.

### Example 4

This example provides a method for FreeRNA spatial transcriptome, comprising the following procedures:
(1) The spatial chip in Example 3 was reacted with 100% formamide at 50 °C, washed with enzyme-free water, and dried for later use;
(2) A FreeRNA hybridization system was prepared according to Table 11 below, uniformly mixed after the preparation was finished, and instantly preserved on ice for later use:

**Table 11**

| Component | Volume (µL) |
|---|---|
| 20× SCC (Sangon, B548110) | 50 |
| Free RNA (528 ng/µL, prepared in-house) | 4 |
| RNase inhibitor (40u/µl Vazyme R301) | 20 |
| Nuclease-free H₂O (Invitrogen AM9939) | 126 |
| Total | 200 |

(3) The FreeRNAhybridization system was dropwise added to the chip treated in step (1), and hybridized at room temperature for 15 min;
(4) A reverse transcription reaction system was prepared according to Table 12 below, uniformly mixed after the preparation was finished, and instantly preserved on ice for later use:

**Table 12**

| Component | Volume (µL) |
|---|---|
| 5× Maxima H RT Buffer (Thermo EP0751) | 20 |
| RNase inhibitor (40u/×L Vazyme R301) | 5 |
| Maxima H (200U/µL Thermo EP0751) | 5 |
| dNTP Mix (10 mM each Vazyme P031) | 10 |
| TSO (100 µM, Sangon) | 5 |
| 5M Betaine (Sigma B0300) | 20 |
| 50% PEG20000 (YEASEN 60300ES60) | 10 |
| Nuclease-free H₂O (Invitrogen AM9939) | 25 |
| Total | 100 |

(5) The free RNA hybridization system was removed, the reverse transcription reaction system was dropwise added to the surface of the chip, and the system was reacted for 2 h at 55 °C and incubated overnight at 42 °C;
(6) A USER enzyme digetion system was prepared according to Table 13 below, uniformly mixed after the preparation was finished, and instantly preserved on ice for later use:

**Table 13**

| Component | Volume (µL) |
|---|---|
| USER (1U/µL NEB M5505L) | 5 |
| 10×rCutsmart (NEB M5505L) | 10 |
| 50% PEG8000 (YEASEN 60300ES60) | 8 |
| Nuclease-free H2O (Invitrogen AM9939) | 77 |
| total | 100 |

(7) The RT MIX reaction system was removed, the USER enzyme digestion system was dropwise added, and the system was reacted for 1 h at 37 °C. The system was mixed by pipetting every 30 min carefully to avoid bubbling;
(8) The USER reaction system was recovered and reacted at 75 °C for 10 min to inactivate the enzyme;
(9) A cDNA amplification system was prepared according to Table 14 below, uniformly mixed after the preparation was finished, and instantly preserved on ice for later use:

**Table 14**

| Component | Volume (µL) |
|---|---|
| 2×KAPA HiFi HotStart ReadyMix (Roche KK2602) | 100 |
| cDNA_F (10 µM, Sangon) | 10 |
| cDNA_R (10 µM, Sangon) | 10 |
| dNTP Mix (10 mM each Vazyme P031) | 20 |
| USER reaction system recovery | 60 |
| Total | 200 |

(10) The PCR reaction was conducted according to the amplification procedures shown in Table 15 below,

**Table 15**

| Temperature | Time | Number of cycles |
|---|---|---|
| 105 °C | Heated lid | 1 |
| 95 °C | 5 min | |
| 98 °C | 20s | 15 |
| 60 °C | 20s | |
| 72 °C | 30s | |
| 72 °C | 3min | 1 |
| 12 °C | Hold | |

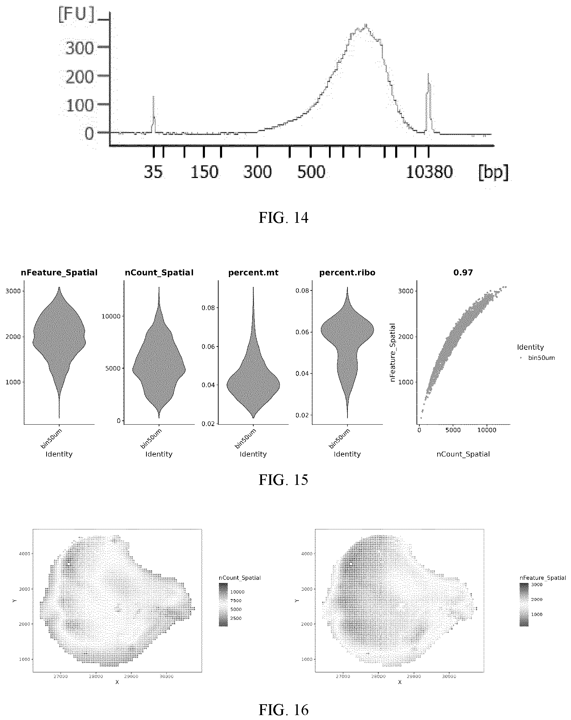

(11) The cDNA products were recovered using 1× VAHTS DNA Clean Beads according to the DNA magnetic bead purification (Vazyme, N411) instructions; the product identification results using the Agilent 2100 system are shown in FIG. 14, which shows that the main peak in the sequence length distribution of cDNA product was about 1500 bp. It can be seen that: the distribution of the full length of cDNA was as expected.
(12) Adapters as shown in Table 16 below were embedded according to the instructions for Tn5 transposase (YEASEN, 14524ES60);

**Table 16**

| Primer | Primer sequence |
|---|---|
| Primer A | 5'-phos-CTGTCTCTTATACACATCT-NH2-3' |
| Primer B | 5'-TCGTCGGCAGCGTCAGATGTGTATAAGAGACAG-3' |

(13) The cDNA product was fragmented using the embedded Tn5 transposase according to the instructions for Tn5 transposase (YEASEN, 14524ES60);
(14) The fragmentation product was subjected to PCR amplification. The system was prepared according to Table 17 below, uniformly mixed after the preparation was finished, and instantly preserved on ice for later use:

**Table 17**

| Component | Volume (µL) |
|---|---|
| 2×KAPA HiFi HotStart ReadyMix (Roche KK2602) | 50 |
| lib_F (10 µM, Sangon) | 5 |
| lib_R (10 µM, Sangon) | 5 |
| dNTP Mix (10 mM each Vazyme P031) | 10 |
| Fragmentation product | 30 |
| Total | 100 |

(15) The PCR reaction was conducted according to the amplification procedures shown in Table 18 below;

**Table 18**

| Temperature | Time | Number of cycles |
|---|---|---|
| 105 °C | Heated lid | 1 |
| 95 °C | 5 min | |
| 98 °C | 20s | 8 |
| 60 °C | 20s | |
| 72 °C | 30s | |
| 72 °C | 3min | 1 |
| 12 °C | Hold | |

(16) The amplified fragmentation product was purified and recovered at 0.5+0.15× according to the DNA magnetic bead purification (Vazyme, N411) instructions.

### Example 5

(1) The product in Example 4 was subjected to high-throughput sequencing, and PE100 sequencing was performed on a sequencing platform (PE denotes paired-end sequencing, and 100 denotes that the length of the sequence of interest is 100 bases). The sequencing data were acquired according to the manual instructions of the sequencing platform;
(2) Data comparison and analysis: 28N and 10 UMI information of the first strand in fq obtained by sequencing was extracted, and the 28N data were compared with fq data obtained by the first sequencing in Example 3 to give positional information, where UMI was used for quantifying the gene expression; the cDNA information of the second strand in fq obtained by sequencing was used for gene alignment, and the results are shown in Table 19 below:

**Table 19**

| BinSize | MeanUM1 | MedianUM1 | MeanGeneNum | MedianGeneNum |
|---|---|---|---|---|
| bin100µm | 21758 | 21069 | 4333 | 4411 |
| bin50µm | 5455 | 5335 | 1976 | 1997 |
| bin25µm | 1363 | 1330 | 757 | 759 |
| bin20µm | 873 | 851 | 539 | 538 |
| bin10µm | 218 | 211 | 173 | 170 |

In the Table, binXµm denotes divided windows having a size of X µm × X µm, for example, bin100µm denotes divided windows having a size of 100 µm × 100 µm; MeanUMI denotes the average number of bonded UMIs, MedianUMI denotes the median number of bonded UMIs, MeanGeneNum denotes the average number of bonded gene types, and MedianGeneNum denotes the median number of bonded gene types.

The bin50µm statistics results are shown in FIG. 15. The ordinates of the first two panels of FIG. 15 denote the number and respectively, the number distribution of bonded genes and UMIs. Panels 3 and 4 show graphs illustrating the log number of the bonded gene types and UMIs, respectively; the abscissa of the last panel denotes the number of bonded UMI and the ordinate denotes the number of bonded gene types. It can be seen that as the number of UMI increased, the identified gene types increased and were eventually saturated.

(3) Taking bin50µm as an example, the sequences of bonded UMIs in the window were detected, and restored to the window according to the positional information corresponding to the sequences, thus giving the left panel of FIG. 16; the Gene types were also restored to the window in accordance with the positional information, thus giving the right panel in FIG. 16. Therefore, the spatial chip provided by the examples of the present application can restore the spatial positional information and achieve Free RNA spatial transcriptomics analysis.

The above description is only for the purpose of illustrating the preferred examples of the present application, and is not intended to limit the scope of the present application. Any modification, equivalent, improvement, and the like made without departing from the spirit and principle of the present application shall fall within the protection scope of the present application.

## Claims

1. A spatial chip, comprising a chip substrate, wherein the chip substrate comprises a first surface provided with a plurality of isolated single-stranded nucleic acid amplification clusters, each of the single-stranded nucleic acid amplification clusters comprises a plurality of single-stranded nucleic acid molecules with identical nucleotide sequences, and nucleotide sequences of different single-stranded nucleic acid amplification clusters are different;
each of the single-stranded nucleic acid amplification clusters has a known physical coordinate set relative to the spatial chip; the single-stranded nucleic acid molecule comprises at least a barcode sequence with a known sequence, and the barcode sequences of each single-stranded nucleic acid amplification clusters are in one-to-one correspondence with the physical coordinate sets.

2. The spatial chip according to claim 1, wherein the number of the amplification clusters on the chip substrate is n, the number of nucleotides constituting the barcode sequence is m, and n and m satisfy: 4^{m} ≥ n; optionally, n is a natural number greater than or equal to 28; and/or, wherein in each of the single-stranded nucleic acid amplification clusters, the copy number of the single-stranded nucleic acid molecules is 10-10⁵; optionally, the area of each of the single-stranded nucleic acid amplification clusters on the first surface is 0.25-100 µm²;
optionally, the distance between adjacent single-stranded nucleic acid amplification clusters is 0.1-10 µm.

3. The spatial chip according to any one of claims 1-2, wherein the single-stranded nucleic acid molecule further comprises a first capture sequence, and the barcode sequence is located at one end of the first capture sequence;
optionally, the first capture sequence is linked to the chip substrate;
optionally, the first capture sequence is linked to the chip substrate by chemical bonding, physical adsorption, electrostatic adsorption, or Van der Waals force;
optionally, a first spacer sequence region is arranged between the first capture sequence and the barcode sequence.

4. The spatial chip according to claim 3, wherein a compound is bonded to the chip substrate, and the first capture sequence is linked to the chip substrate via the compound;
optionally, the chip substrate is provided with a first group, the compound is provided with a second group, the first group and the second group are a chemically reactive group set, and the compound is bonded to the chip substrate via the group set;
optionally, the group set is selected from an amino-carboxylic acid set, an amino-ester set, an amino-halogen set, an amino-formyl set, an amino-epoxy group set, a sulfydryl-imino set, an azido-alkynyl set, an azido-alkenyl set, and a cycloalkenyl-tetrazinyl set;
optionally, the compound is selected from one or more of an amino compound, a carboxylate compound, succinimide or an analog thereof, a pentafluorophenol ester or an analog thereof, an aryl halide, maleimide or
an analog thereof, an epoxy compound, a formyl-containing compound, an azido-containing compound, norbornene or an analog thereof, a DBCO or an analog thereof, and a tetrazine and an analog thereof.

5. The spatial chip according to claim 3, wherein the chip substrate is provided with a first reactant, the first capture sequence is provided with a second reactant, the first reactant and the second reactant are reactive, and the first capture sequence is linked to the chip substrate via the first reactant and the second reactant; optionally, the first reactant is different from the second reactant, and the first reactant and the second reactant are each independently selected from an antigen and an antibody, or the first reactant and the second reactant are each independently selected from biotin and streptavidin.

6. The spatial chip according to claim 3, wherein the single-stranded nucleic acid molecule further comprises a second capture sequence, and the second capture sequence is linked to the barcode sequence at the end distal to the first capture sequence;
optionally, a PolyN sequence is arranged between the second capture sequence and the barcode sequence, and N is selected from one of A or a derivative thereof, T/U or a derivative thereof, G or a derivative thereof, and C or a derivative thereof;
optionally, a second spacer sequence region is arranged between the second capture sequence and the barcode sequence; preferably wherein a cleavable site is arranged in the first capture sequence and the second capture sequence;
optionally, the cleavable site is at least one of a photocleavable site, a chemically cleavable site, or an enzyme-cleavable site;
optionally, when the cleavable site is an enzyme-cleavable site, the barcode sequence does not comprise a sequence identical to the cleavable site.

7. The spatial chip according to any one of claims 1-6, wherein the chip substrate has a planar surface, a spherical surface, or an irregular surface;
optionally, the chip substrate is provided with a plurality of micro/nano structures arranged according to a preset rule, and the single-stranded nucleic acid amplification cluster is immobilized on a surface of the micro/nano structure;
optionally, the micro/nano structure is a nano depression and/or a nano protrusion;
optionally, the micro/nano structure has a circular, elliptical, polygonal, or irregular cross-sectional profile;
optionally, the minimum distance between adjacent micro/nano structures is 10 nm to 100 µm, and the maximum size of a shape of the micro/nano structure on a surface of the chip substrate is 10 nm to 100 µm; and/or
wherein the first surface is provided with one or more markers;
optionally, the marker is one of a letter, a number and a graphic, or a combination thereof;
optionally, the marker includes a directional marker and a regional marker;
optionally, a plurality of regional markers are present and arranged on the first surface and close to a circumference of the chip substrate;
optionally, the chip substrate has a polygonal shape, and the regional markers are uniformly distributed in a direction parallel to each side of the polygon; or
the chip substrate is circular or elliptical, and the regional markers are uniformly distributed along a track close to the circumference;
optionally, the marker further includes a chip serial number marker; preferably wherein the chip substrate comprises a second surface arranged opposite to the first surface;
optionally, the second surface is provided with a chip label; the chip label is configured for recording the physical coordinate set of each of the amplification clusters on the chip substrate and a sequence of the barcode sequence region at each of the physical coordinate sets;
optionally, the chip label is a QR code or a barcode.

8. The spatial chip according to any one of claims 1-7, wherein the spatial chip comprises two or more chip substrates, and the chip substrates are immobilized on a surface of a solid carrier in a preset arrangement;
optionally, the chip substrate has known positional information relative to the solid carrier;
optionally, a surface of the solid carrier is provided with a chip label; the chip label is configured for recording the physical coordinate set of each of the amplification clusters on the chip substrate and a sequence of the barcode sequence region at each of the physical coordinate sets;
optionally, the positional information is acquired by the chip serial number marker of the first surface;
optionally, a glue layer is arranged between the chip substrate and the solid carrier;
optionally, the glue layer covers part or all of the second surface;
optionally, the glue layer has a light transmittance of greater than or equal to 90%;
optionally, the chip substrate is arrayed on the surface of the solid carrier;
optionally, the distance between the circumference of the chip substrate and the circumference of the solid carrier is greater than or equal to 1 mm;
optionally, the distance between adjacent chip substrates is greater than or equal to 1 mm;
optionally, the solid carrier has a light transmittance of greater than or equal to 90%.

9. A method for preparing a spatial chip, comprising:
synthesizing a first single-stranded nucleotide library with a barcode sequence, wherein nucleotide sequences of different first single-stranded nucleotide libraries are different, and the barcode sequences of the first single-stranded nucleotide libraries are different from each other;
immobilizing the first single-stranded nucleotide library on a surface of a solid substrate, and amplifying the first single-stranded nucleotide library to give an amplification cluster;
linearizing the amplification cluster to give a single-stranded nucleic acid amplification cluster; and
sequencing the single-stranded nucleic acid amplification cluster to acquire the barcode sequence of each of the single-stranded nucleic acid amplification clusters and a physical coordinate set of each of the single-stranded nucleic acid amplification clusters on a surface of the chip substrate, and determining information that the barcode sequences of the first single-stranded nucleotide library are in one-to-one correspondence with the physical coordinate sets, so as to give the spatial chip.

10. The method according to claim 9, wherein the number of the amplification clusters on the surface of the solid substrate is n, the number of nucleotides constituting the barcode sequence is m, and n and m satisfy: 4^{m} ≥ n;
optionally, n is a natural number greater than or equal to 28;
optionally, the area of each of the amplification clusters is 0.25-100 µm²;
optionally, in each of the amplification clusters, the copy number of the nucleic acid molecules is 10-10⁵;
optionally, the distance between adjacent nucleic acid amplification clusters is 0.1-10 µm.

11. The method according to claim 10, wherein the first single-stranded nucleotide library further comprises a first capture sequence, and the barcode sequence is located at one end of the first capture sequence;
optionally, in the step of immobilizing the first single-stranded nucleotide library on the surface of the solid substrate, the first capture sequence is bonded to the surface of the solid substrate;
optionally, the first capture sequence is linked to the surface of the chip substrate by chemical bonding, physical adsorption, electrostatic adsorption, or Van der Waals force;
optionally, a compound is bonded to the surface of the solid substrate, and the first capture sequence is linked to the chip substrate via the compound;
optionally, the surface of the solid substrate is provided with a first group, the compound is provided with a second group, the first group and the second group are a chemically reactive group set, and the compound is bonded to the surface of the chip substrate via the group set;
optionally, the group set is selected from an amino-carboxylic acid set, an amino-ester set, an amino-halogen set, an amino-formyl set, an amino-epoxy group set, a sulfydryl-imino set, an azido-alkynyl set, an azido-alkenyl set, and a cycloalkenyl-tetrazinyl set;
optionally, the compound is selected from one or more of an amino compound, a carboxylate compound, succinimide or an analog thereof, a pentafluorophenol ester or an analog thereof, an aryl halide, maleimide or an analog thereof, an epoxy compound, a formyl-containing compound, an azido-containing compound, norbornene or an analog thereof, a DBCO or an analog thereof, and a tetrazine and an analog thereof;
optionally, the surface of the solid substrate is provided with a first reactant, the first capture sequence is provided with a second reactant, the first reactant and the second reactant are reactive, and the first capture sequence is linked to the surface of the solid substrate via the first reactant and the second reactant;
optionally, the first reactant is different from the second reactant, and the first reactant and the second reactant are each independently selected from an antigen and an antibody, or the first reactant and the second reactant are each independently selected from biotin and streptavidin;
optionally, the first single-stranded nucleotide library further comprises a second capture sequence, and the second capture sequence is linked to the barcode sequence at the end distal to the first capture sequence;
optionally, a PolyN sequence is arranged between the second capture sequence and the barcode sequence, and N is selected from one of A or a derivative thereof, T/U or a derivative thereof, G or a derivative thereof, and C or a derivative thereof;
optionally, a cleavable site is arranged in the first capture sequence and the second capture sequence;
optionally, the cleavable site is at least one of a photocleavable site, a chemically cleavable site, or an enzyme-cleavable site;
optionally, when the cleavable site is an enzyme-cleavable site, the barcode sequence does not comprise a sequence identical to the cleavable site;
optionally, the step of linearizing the amplification cluster comprises: adding a restriction endonuclease, and digesting the amplification cluster at an enzyme-cleavable site.

12. The method according to any one of claims 9-11, wherein the surface of the solid substrate is a planar surface, a spherical surface, or a non-planar surface formed by a dendrimer;
optionally, the surface of the solid substrate is provided with a nanowell or a nanopore, and the first single-stranded nucleotide library is immobilized in the nanowell or the nanopore.

13. The method according to any one of claims 9-12, wherein the solid substrate is derived from a chip mother substrate having an area 2 or more times a surface area of the spatial chip, and the method further comprises, before the spatial chip is given: cutting a surface of the chip mother substrate into a chip substrate with a preset size;
optionally, the step of cutting a surface of the chip mother substrate into a chip substrate with a preset size comprises:
determining one or more preset chip regions on the surface of the chip mother substrate according to a preset shape and the preset size, and marking the preset chip region;
separating the preset chip region from the chip mother substrate to give the spatial chip, wherein the spatial chip comprises the chip substrate, the chip substrate is provided with a first surface and a second surface opposite to the first surface, and the single-stranded nucleic acid amplification cluster is located on the first surface;
optionally, the method further comprises: fixing one or more chip substrates on a surface of a solid carrier in a preset arrangement; preferably wherein marking the preset chip region comprises:
printing a marker on a surface of the preset chip region by laser printing;
optionally, the marking comprises forming the marker on the first surface;
optionally, the marker includes a directional marker, a regional marker, and a chip serial number marker;
optionally, a plurality of regional markers are present and arranged on the first surface and close to a circumference of the first substrate;
optionally, the marker is one of a letter, a number and a graphic, or a combination thereof;
optionally, separating the preset chip region from the chip mother substrate comprises:
cutting and separating the preset chip region from the chip mother substrate by laser according to a preset cutting line;
optionally, the distance between adjacent preset cutting lines is greater than or equal to 2 mm;
optionally, the method further comprises:
fixing the chip substrate on one surface of the solid carrier;
optionally, a surface of the solid carrier is provided with a chip label; the chip label is configured for recording the physical coordinate set of each of the amplification clusters on the chip substrate and a sequence of the barcode sequence region at each of the physical coordinate sets; or alternatively, the second surface is provided with a chip label; the chip label is configured for recording the physical coordinate set of each of the amplification clusters on the chip substrate and a sequence of the barcode sequence region at each of the physical coordinate sets;
optionally, the chip label is a QR code or a barcode;
optionally, the second surface is bonded to the solid carrier, and the chip substrate has known positional information relative to the solid carrier;
optionally, a glue layer is arranged between the chip substrate and the solid carrier;
optionally, the glue layer covers part or all of the second surface;
optionally, the glue layer has a light transmittance of greater than or equal to 90%;
optionally, the chip substrate is arrayed on the solid carrier;
optionally, the distance between the circumference of the chip substrate and the circumference of the solid carrier is greater than or equal to 1 mm;
optionally, the distance between adjacent chip substrates is greater than or equal to 1 mm.

14. Use of the spatial chip according to any one of claims 1-8 or a spatial chip prepared by the method according to any one of claims 9-13 in the field of spatial omics.

15. The use according to claim 14, wherein the use is a method for spatial reverse transcription comprising:
providing the spatial chip and a second single-stranded nucleotide library, wherein the spatial chip comprises a chip substrate; at least one surface of the chip substrate is provided with a plurality of isolated single-stranded nucleic acid amplification clusters; single-stranded nucleic acid molecules in the single-stranded nucleic acid amplification cluster have identical nucleotide sequences, and nucleotide sequences of different single-stranded nucleic acid amplification clusters are different; the single-stranded nucleic acid amplification cluster has a known physical coordinate set relative to the spatial chip, and the single-stranded nucleic acid molecule comprises a barcode sequence with a known sequence and a first capture sequence and a second capture sequence respectively linked to the two ends of the barcode sequence; the first capture sequence is linked to a surface of the chip substrate, and the barcode sequences of each single-stranded nucleic acid amplification clusters are in one-to-one correspondence with the physical coordinate sets; the second single-stranded nucleotide library comprises a first base sequence, a second base sequence and a capture part that are linked in sequence; the first base sequence is a known sequence complementary with the second capture sequence, the second base sequence is a label sequence, the capture part is a primer sequence, and first base sequences of the nucleic acid in the second single-stranded nucleotide library are identical, but the label sequences are different;
contacting a cell or tissue sample of interest with the spatial chip and the second single-stranded nucleotide library to bond RNA in the cell or tissue sample of interest on the spatial chip via the second single-stranded nucleotide library;
cleaving the spatial chip, and collecting a cleavage product; and
sequencing the cleavage product, and analyzing obtained sequencing data;
optionally, when the cell or tissue sample of interest is a tissue sample, the tissue sample is a tissue section;
optionally, the tissue section has a thickness of less than or equal to 50 µm;
optionally, the step of contacting the cell or tissue sample of interest with the spatial chip and the second single-stranded nucleotide library comprises:
contacting the cell or tissue sample of interest with the spatial chip, and then adding the second single-stranded nucleotide library;
optionally, analyzing the obtained sequencing data comprises:
on the basis of the sequencing data, acquiring positional information in the spatial chip of the barcode sequence in the cleavage product; and
on the basis of the positional information, performing spatial restoration on the cleavage product to achieve spatial reverse transcription analysis;
or wherein the use is a method for ATAC sequencing comprising:
providing the spatial chip and a second single-stranded nucleotide library, wherein the spatial chip comprises a chip substrate; at least one surface of the chip substrate is provided with a plurality of isolated single-stranded nucleic acid amplification clusters; single-stranded nucleic acid molecules in the single-stranded nucleic acid amplification cluster have identical nucleotide sequences, and nucleotide sequences of different single-stranded nucleic acid amplification clusters are different; the single-stranded nucleic acid amplification cluster has a known physical coordinate set relative to the spatial chip, and the single-stranded nucleic acid molecule comprises a barcode sequence with a known sequence and a first capture sequence and a second capture sequence respectively linked to the two ends of the barcode sequence; the first capture sequence is linked to a surface of the chip substrate, and the barcode sequences of each single-stranded nucleic acid amplification clusters are in one-to-one correspondence with the physical coordinate sets; the second single-stranded nucleotide library comprises a first base sequence, a second base sequence, and a capture part; the first base sequence is a known sequence, the second base sequence is a label sequence, and first base sequences of the nucleotides in the second single-stranded nucleotide library are identical, but the label sequences are different; the capture part is a transposase, and the first base sequence and the second base sequence are located in the transposase;
contacting a cell or tissue sample of interest with the spatial chip and the second single-stranded nucleotide library to bond ATAC in the cell or tissue sample of interest on the spatial chip via the second single-stranded nucleotide library;
cleaving the spatial chip, and collecting a cleavage product; and
sequencing the cleavage product, and analyzing obtained sequencing data;
optionally, when the cell or tissue sample of interest is a tissue sample, the tissue sample is a tissue section;
optionally, the tissue section has a thickness of less than or equal to 50 µm;
optionally, the step of contacting the cell or tissue sample of interest with the spatial chip and the second single-stranded nucleotide library comprises:
contacting the cell or tissue sample of interest with the spatial chip, and then adding the second single-stranded nucleotide library;
optionally, analyzing the obtained sequencing data comprises:
on the basis of the sequencing data, acquiring positional information in the spatial chip of the barcode sequence in the cleavage product; and
on the basis of the positional information, performing spatial restoration on the cleavage product to achieve ATAC sequencing analysis;
or wherein the use is a method for spatial protein analysis comprising:
providing the spatial chip and a second single-stranded nucleotide library, wherein the spatial chip comprises a chip substrate; at least one surface of the chip substrate is provided with a plurality of isolated single-stranded nucleic acid amplification clusters; single-stranded nucleic acid molecules in the single-stranded nucleic acid amplification cluster have identical nucleotide sequences, and nucleotide sequences of different single-stranded nucleic acid amplification clusters are different; the single-stranded nucleic acid amplification cluster has a known physical coordinate set relative to the spatial chip, and the single-stranded nucleic acid molecule comprises a barcode sequence with a known sequence and a first capture sequence and a second capture sequence respectively linked to the two ends of the barcode sequence; the first capture sequence is linked to a surface of the chip substrate; the second single-stranded nucleotide library comprises a first base sequence, a second base sequence and a capture part that are linked in sequence; the first base sequence is a known sequence complementary with the second capture sequence, the second base sequence is a label sequence, and first base sequences of the nucleotides in the second single-stranded nucleotide library are identical, but the label sequences are different;
contacting a cell or tissue sample of interest with the spatial chip and the second single-stranded nucleotide library to bond a target having a binding capacity with the capture part in the sample of interest on the spatial chip via the second single-stranded nucleotide library;
cleaving the spatial chip, and collecting a cleavage product; and
sequencing the cleavage product, and analyzing obtained sequencing data;
optionally, the capture part is a nucleic acid aptamer, a protein, an enzyme, or an antibody;
optionally, the sample of interest is the cell or tissue sample of interest;
optionally, when the cell or tissue sample of interest is a tissue sample, the tissue sample is a tissue section;
optionally, the tissue section has a thickness of less than or equal to 50 µm;
optionally, the step of contacting the cell or tissue sample of interest with the spatial chip and the second single-stranded nucleotide library comprises:
contacting the cell or tissue sample of interest with the spatial chip, and then adding the second single-stranded nucleotide library;
optionally, analyzing the obtained sequencing data comprises:
on the basis of the sequencing data, acquiring positional information in the spatial chip of the barcode sequence in the cleavage product; and on the basis of the positional information, performing spatial restoration on the cleavage product to achieve spatial protein analysis.
